# EUROPEAN PATENT SPECIFICATION

(11) **EP 3 229 779 B1**
(45) Date of publication and mention of the grant of the patent: **03.02.2021**
(21) Application number: 15820375.2
(22) Date of filing: 10.12.2015
(51) Int. Cl.: A61K 9/00, A61K 47/10

(54) **HYDROGEL DRUG DELIVERY IMPLANTS**
HYDROGEL-WIRKSTOFFFREISETZUNGSIMPLANTATE
IMPLANTS D'ADMINISTRATION DE MÉDICAMENT À BASE D'HYDROGEL

(30) Priority: 10.12.2014 US 201462089994 P
(43) Date of publication of application: 18.10.2017
(60) Divisional application: 20214440.8
(73) Proprietor: Incept, LLC, Lexington, MA 02420 (US)
(72) Inventor: JARRETT, Peter, Lexington, MA 02420 (US); EL-HAYEK, Rami, Norwood, MA 02062 (US); SAWHNEY, Amarpreet S., Lexington, MA 02420 (US)
(74) Representative: dompatent von Kreisler Selting Werner - Partnerschaft von Patent- und Rechtsanwälten mbB
(86) International application number: PCT/US2015/064975
(87) International publication number: WO 2016/094646

(56) References cited:
- US-A1- 2002 138 154
- US-A1- 2009 017 097
- US-A1- 2013 071 462
- US-A1- 2013 156 752

## Description

### CROSS REFERENCE TO RELATED APPLICATIONS

This patent application claims priority to U.S. Provisional Application No. 62/089,994 filed December 10, 2014.

### TECHNICAL FIELD

The technical field is related to compositions for treating the body, and includes pharmaceutically acceptable implant systems comprising a collection of pharmaceutically acceptable, covalently-crosslinked hydrogel particles having therapeutic agents that are disposed in a surrounding hydrogel.

### BACKGROUND

Implants that deliver drugs over time in a therapeutically effective dosage are useful in many fields. The science of controlled drug release is diverse from a standpoint of both range of scientific disciplines it encompasses and the range of its applications.

### BRIEF DESCRIPTION OF THE DRAWINGS

Fig. 1 is a schematic of a process of making a hydrogel encapsulating hydrogel particles that contain a therapeutic agent;
Fig. 2 is a schematic showing release of the therapeutic agent from the embodiment of Fig. 1;
Fig. 3 depicts an eye with hydrogels such as the hydrogels of Fig. 1 in place in an eye;
Fig. 4 is a plot of data of experimental results; and
Fig. 5 depicts two curves showing a delay in release caused by a coating.

### DETAILED DESCRIPTION

Hydrogel particles are used for controlled release of therapeutic agents to deliver them over time. In general, the particles may be placed at a site where delivery of the agent is desired and the agent is released as the hydrogel reacts with the physiological fluids at the site. In areas such as an eye, a large concentration of the agent in the hydrogel is generally desirable since space in, on, or inside the eye is limited. Even in sites where space is not as limited, keeping the volume of the treatment close to its minimum necessary volume is to be expected to optimize the delivery system. But the inventors have found that it is helpful to add a certain amount of extra hydrogel to these systems as disclosed in claim 1 and in claim 9 (the kit for preparing the system of claim 1); the hydrogel is preferably free of the agent that is to be delivered. The invention is as defined in claim 1 and discloses a biomedical sustained release system for use as a medicament in the treatment of an eye disease, especially as an eye implant comprising a collection of particles that comprise a first biodegradable material that is a hydrogel or a xerogel made of hydrophilic materials and a therapeutic agent, with the particles, before biodegradation of the first material, having a rate of release for the therapeutic agent as measured in physiological solution, and a second material that is a hydrogel or xerogel that at least partially coats the collection of particles wherein the hydrogel made of the second material delays the rate of release of the agent by no more than 20% as measured at the 50% w/w release of the agent.

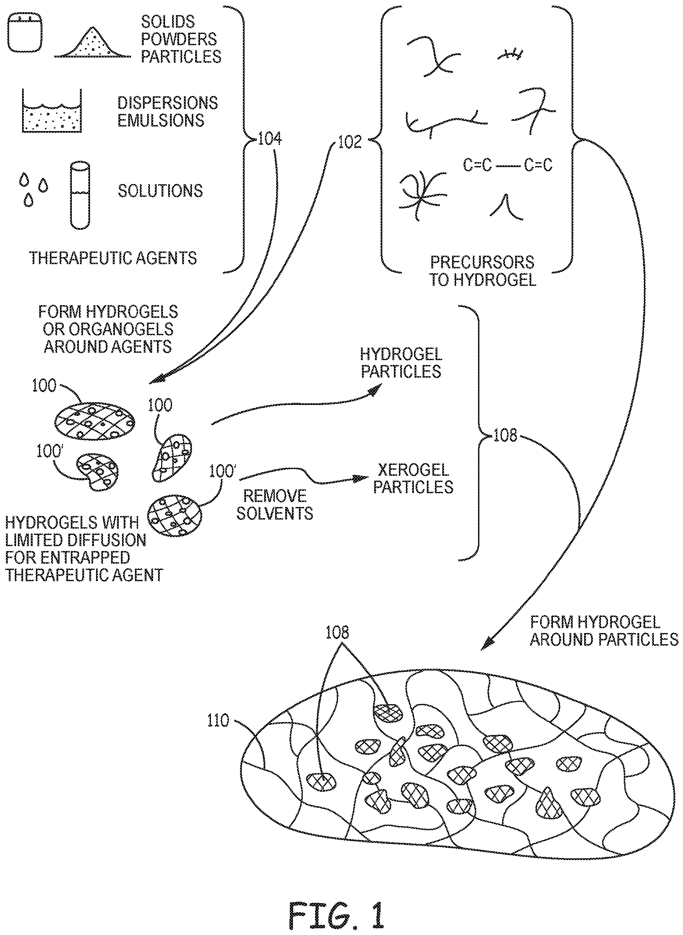

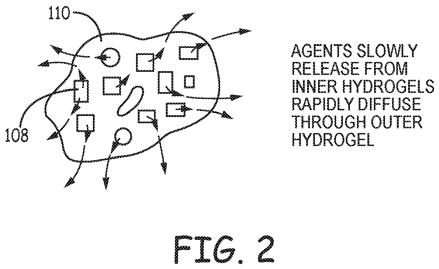

By way of example, referring to Figs. 1-2, hydrogels 100 or organogels 100' are formed by crosslinking precursors 102 around a therapeutic agent 104. Hydrogels 100 or organogels 100' may be formed as particles or as a larger hydrogel/organogel that is processed into particulates. Hydrogels 100 or organogels 100' may be used directly, made into xerogels, or otherwise processed to form particles 108, which are hydrogels or xerogels. A hydrogel 110 (or an organogel) is formed from precursors 102 around particles 108. Hydrogel 110 (or the organogel) can be made into a xerogel that is later rehydrated. Agents in hydrogel particles 108 are released when the hydrogel is in aqueous solution, with any xerogels becoming hydrogels when exposed to the aqueous solution. The hydrogel particles provide for diffusion of therapeutic agents 104 outwards into hydrogel 110. Hydrogel 110 does not change the rate of release, or provides a minimal change in the rate of release of the agent. Briefly, in use, hydrogels 108 are formed ex vivo and hydrogel 110 is formed ex vivo or may be formed in situ. The term in situ means at the site of intended use wherein the hydrogel is to be used, e.g., on a tissue of the patient. The hydrogels interact with physiological fluids in the body and release the agents over time.

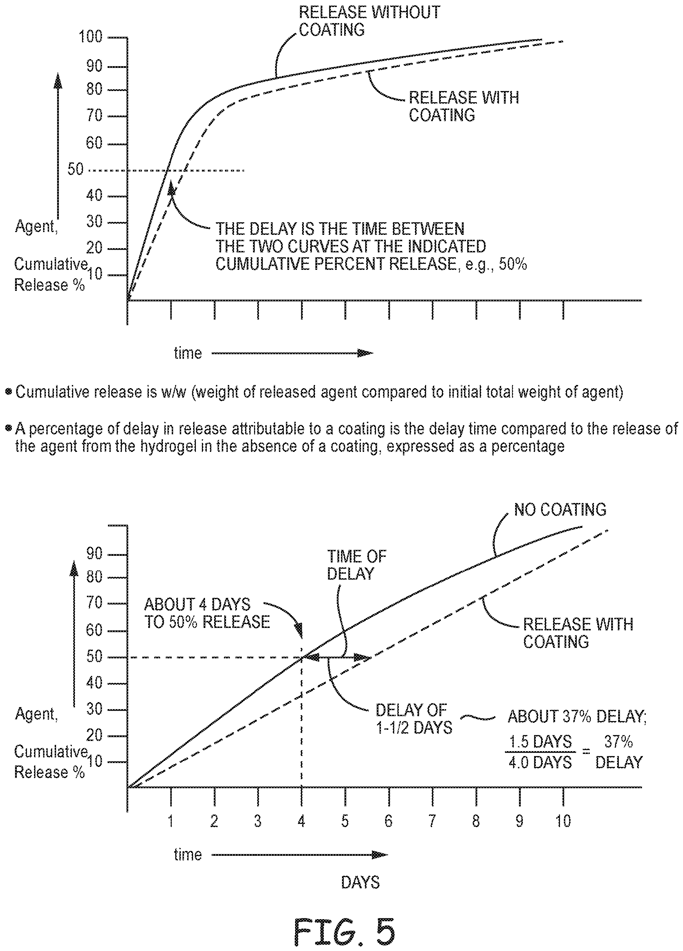

Fig. 5 depicts two sets of controlled release profiles. The coated samples, indicated in dashed lines, delay release of the agent. The delay is the time between the release profiles for coated versus non-coated samples. The percentage delay may be calculated at a given cumulative release percentage by measuring the delay at that point divided by the time required for the cumulative release from the non-coated sample. These are hypothetical curves. Actual data can be expected to show variations in the profiles; artisans, however, can readily generate an amount of data to accurately compare coated and non-coated samples for determining an accurate measurement.

There are various ways to quantify the similarity between coated versus non-coated release profiles. In general, it may be helpful to look at the profile across a limited range of the cumulative release percentage since release at the earliest and latest parts of the curves can involve only a small portion of the total released amount. Accordingly, options include assessing release rate at a given cumulative percentage of released agent, e.g., at 50%. Alternatives could be some other point, e.g., between 10 and 90 percent; artisans will immediately appreciate that all ranges and values within this range are contemplated and supported, e.g., 20%, 25%, 33%, 60%, 67%, and so forth. Another option is to measure the delay (maximum delay, average delay, mean delay) across an entire range, e.g., from 10% to 90%; artisans will immediately appreciate that all ranges and values within this range are contemplated and supported, e.g., from 20% to 60%, from 10% to 50%, from 33% to 67%, from 15% to 95%.

In further embodiments, a first material comprising a hydrogel or a xerogel made of hydrophilic materials that will become a hydrogel is coated with a second material that is a hydrogel, a xerogel, or precursors that will become a hydrogel by crosslinking with each other upon exposure to physiological or other aqueous solution. The coating of precursors may be dry, deposited as a powder, a melt, or mixed with binders or other excipients, e.g., plasticizers, salts, lubricants, and so forth.

### Precursor materials

The hydrogels are made from precursors. Precursors are chosen in consideration of the properties that are desired for the resultant hydrogel. There are various suitable precursors for use in making the hydrogels and/or the organogels. The term precursor refers to those molecules crosslinked to form the hydrogel or organogel matrix. While other materials might be present in the hydrogel or organogel, such as therapeutic agents or fillers, they are not precursors. The term matrix is applicable for hydrogels, organogels, and xerogels. Such matrices include matrices with a solvent content of more than about 20% w/w; artisans will immediately appreciate that all the ranges and values within the explicitly stated range is contemplated, including 20% to 99%, 80% to 95%, at least 50%, and so forth, with the percentages being w/w and the solvent being water for hydrogels and the liquid organic for organogels.

Precursors may be dissolved in an organic solvent to make an organogel. An organogel is a non-crystalline, non-glassy solid material composed of a liquid organic phase entrapped in a three-dimensionally cross-linked network. The liquid can be, for example, an organic solvent, mineral oil, or vegetable oil. The solubility and dimensions of the solvent are important characteristics for the elastic properties and firmness of the organogel. Alternatively, the precursor molecules may themselves be capable of forming their own organic matrix, eliminating the need for a tertiary organic solvent. Removal of the solvent (if used) from the organogel provides a xerogel, a dried gel. The xerogels are formed by, for example, freeze drying, may have a high porosity (at least about 20%, a large surface area, and a small pore size. Xerogels made with hydrophilic materials form hydrogels when exposed to aqueous solutions. High porosity xerogels hydrate more quickly than more dense xerogels. Hydrogels are materials that do not dissolve in water and retain a significant fraction (more than 20%) of water within their structure. In fact, water contents in excess of 90% are often known. Hydrogels may be formed by crosslinking water soluble molecules to form networks of essentially infinite molecular weight. Hydrogels with high water contents are typically soft, pliable materials. Hydrogels and drug delivery systems as described in U.S. Publication Nos. 2009/0017097, 2011/0142936 and 2012/0071865 may be adapted for use with the materials and methods herein by following the guidance provided herein. Ophthalmic implant as described in US2013/071462 are made of lipophilic particles with drugs entrapped in a second material being an hydrogel.

Organogels and hydrogels may be formed from natural, synthetic, or biosynthetic polymers. Natural polymers may include glycosminoglycans, polysaccharides, and proteins. Some examples of glycosaminoglycans include dermatan sulfate, hyaluronic acid, the chondroitin sulfates, chitin, heparin, keratan sulfate, keratosulfate, and derivatives thereof. In general, the glycosaminoglycans are extracted from a natural source and purified and derivatized. However, they also may be synthetically produced or synthesized by modified microorganisms such as bacteria. These materials may be modified synthetically from a naturally soluble state to a partially soluble or water swellable or hydrogel state. This modification may be accomplished by various well-known techniques, such as by conjugation or replacement of ionizable or hydrogen bondable functional groups such as carboxyl and/or hydroxyl or amine groups with other more hydrophobic groups.

For example, carboxyl groups on hyaluronic acid may be esterified by alcohols to decrease the solubility of the hyaluronic acid. Such processes are used by various manufacturers of hyaluronic acid products (such as Genzyme Corp., Cambridge, MA) to create hyaluronic acid based sheets, fibers, and fabrics that form hydrogels. Other natural polysaccharides, such as carboxymethyl cellulose or oxidized regenerated cellulose, natural gum, agar, agrose, sodium alginate, carrageenan, fucoidan, furcellaran, laminaran, hypnea, eucheuma, gum arabic, gum ghatti, gum karaya, gum tragacanth, locust beam gum, arbinoglactan, pectin, amylopectin, gelatin, hydrophilic colloids such as carboxymethyl cellulose gum or alginate gum crosslinked with a polyol such as propylene glycol, and the like, also form hydrogels upon contact with aqueous surroundings.

Synthetic organogels or hydrogels may be biostable or biodegradable. Examples of biostable hydrophilic polymeric materials are poly(hydroxyalkyl methacrylate), poly(electrolyte complexes), poly(vinylacetate) cross-linked with hydrolysable or otherwise degradable bonds, and water-swellable N-vinyl lactams. Other hydrogels include hydrophilic hydrogels known as CARBOPOL®, an acidic carboxy polymer (Carbomer resins are high molecular weight, allylpentaerythritol-crosslinked, acrylic acid-based polymers, modified with C10-C30 alkyl acrylates), polyacrylamides, polyacrylic acid, starch graft copolymers, acrylate polymer, ester cross-linked polyglucan. Such hydrogels are described, for example, in U.S. Patent No. 3,640,741 to Etes, U.S. Patent No. 3,865,108 to Hartop, U.S. Patent No. 3,992,562 to Denzinger et al., U.S. Patent No. 4,002,173 to Manning et al., U.S. Patent No. 4,014,335 to Arnold and U.S. Patent No. 4,207,893 to Michaels.

Hydrogels and organogels may be made from precursors. The precursors are crosslinked with each other. Crosslinks can be formed by covalent bonds or physical bonds. Examples of physical bonds are ionic bonds, hydrophobic association of precursor molecule segments, and crystallization of precursor molecule segments. The precursors can be triggered to react to form a crosslinked hydrogel. The precursors can be polymerizable and include crosslinkers that are often, but not always, polymerizable precursors. Polymerizable precursors are thus precursors that have functional groups that react with each other to form matrices and/or polymers made of repeating units. Precursors may be polymers.

Some precursors thus react by chain-growth polymerization, also referred to as addition polymerization, and involve the linking together of monomers incorporating double or triple chemical bonds. These unsaturated monomers have extra internal bonds which are able to break and link up with other monomers to form the repeating chain. Monomers are polymerizable molecules with at least one group that reacts with other groups to form a polymer. A macromonomer (or macromer) is a polymer or oligomer that has at least one reactive group, often at the end, which enables it to act as a monomer; each macromonomer molecule is attached to the polymer by reaction the reactive group. Thus macromonomers with two or more monomers or other functional groups tend to form covalent crosslinks. Addition polymerization is involved in the manufacture of, e.g., polypropylene or polyvinyl chloride. One type of addition polymerization is living polymerization.

Some precursors thus react by condensation polymerization that occurs when monomers bond together through condensation reactions. Typically these reactions can be achieved through reacting molecules incorporating alcohol, amine or carboxylic acid (or other carboxyl derivative) functional groups. When an amine reacts with a carboxylic acid an amide or peptide bond is formed, with the release of water. Some condensation reactions follow a nucleophilic acyl substitution, e.g., as in U.S. Patent No. 6,958,212. Some precursors react by a chain growth mechanism. Chain growth polymers are defined as polymers formed by the reaction of monomers or macromonomers with a reactive center. A reactive center is a particular location within a chemical compound that is the initiator of a reaction in which the chemical is involved. In chain-growth polymer chemistry, this is also the point of propagation for a growing chain. The reactive center is commonly radical, anionic, or cationic in nature, but can also take other forms. Chain growth systems include free radical polymerization, which involves a process of initiation, propagation and termination. Initiation is the creation of free radicals necessary for propagation, as created from radical initiators, e.g., organic peroxide molecules. Termination occurs when a radical reacts in a way that prevents further propagation. The most common method of termination is by coupling where two radical species react with each other forming a single molecule. Some precursors react by a step growth mechanism, and are polymers formed by the stepwise reaction between functional groups of monomers. Most step growth polymers are also classified as condensation polymers, but not all step growth polymers release condensates. Monomers may be polymers or small molecules. A polymer is a high molecular weight molecule formed by combining many smaller molecules (monomers) in a regular pattern. Molecular weights for polymers refer to weight average molecular weights unless otherwise specified. Oligomers are polymers having less than about 20 monomeric repeat units. A small molecule generally refers to a molecule that is less than about 2000 Daltons. The precursors may thus be small molecules, such as acrylic acid or vinyl caprolactam, larger molecules containing polymerizable groups, such as acrylate-capped polyethylene glycol (PEG-diacrylate), or other polymers containing ethylenically-unsaturated groups, such as those of U.S. Patent No. 4,938,763 to Dunn et al, U.S. Patent Nos. 5,100,992 and 4,826,945 to Cohn et al, or U.S. Patent Nos. 4,741,872 and 5,160,745 to DeLuca et al.

To form covalently crosslinked hydrogels, the precursors must be covalently crosslinked together. In general, polymeric precursors are polymers that will be joined to other polymeric precursors at two or more points, with each point being a linkage to the same or different polymers. Precursors with at least two reactive centers (for example, in free radical polymerization) can serve as crosslinkers since each reactive group can participate in the formation of a different growing polymer chain. In the case of functional groups without a reactive center, among others, crosslinking requires three or more such functional groups on at least one of the precursor types. For instance, many electrophilic-nucleophilic reactions consume the electrophilic and nucleophilic functional groups so that a third functional group is needed for the precursor to form a crosslink. Such precursors thus may have three or more functional groups and may be crosslinked by precursors with two or more functional groups. A crosslinked molecule may be crosslinked via an ionic or covalent bond, a physical force, or other attraction. A covalent crosslink, however, will typically offer stability and predictability in reactant product architecture.

In some embodiments, each precursor is multifunctional, meaning that it comprises two or more electrophilic or nucleophilic functional groups, such that a nucleophilic functional group on one precursor may react with an electrophilic functional group on another precursor to form a covalent bond. At least one of the precursors comprises more than two functional groups, so that, as a result of electrophilic-nucleophilic reactions, the precursors combine to form crosslinked polymeric products.

The precursors may have biologically inert and hydrophilic portions, e.g., a core. In the case of a branched polymer, a core refers to a contiguous portion of a molecule joined to arms that extend from the core, with the arms having a functional group, which is often at the terminus of the branch. A hydrophilic molecule, e.g., a precursor or precursor portion, has a solubility of at least 1 g/100 mL in an aqueous solution. A hydrophilic portion may be, for instance, a polyether, for example, polyalkylene oxides such as polyethylene glycol (PEG), polyethylene oxide (PEO), polyethylene oxide-co-polypropylene oxide (PPO), co-polyethylene oxide block or random copolymers, and polyvinyl alcohol (PVA), poly (vinyl pyrrolidinone) (PVP), poly (amino acids, dextran, or a protein. The precursors may have a polyalkylene glycol portion and may be polyethylene glycol based, with at least about 80% or 90% by weight of the polymer comprising polyethylene oxide repeats. The polyethers and more particularly poly (oxyalkylenes) or poly (ethylene glycol) or polyethylene glycol are generally hydrophilic. As is customary in these arts, the term PEG is used to refer to PEO with or without hydroxyl end groups.

A precursor may also be a macromolecule (or macromer), which is a molecule having a molecular weight in the range of a thousand to many millions. The hydrogel or organogel however, may be made with at least one of the precursors as a small molecule of about 1000 Da or less (alternatively: 2000 Da or less). The macromolecule, when reacted in combination with a small molecule (of about 1000 Da or less / 200 Da or less), is preferably at least five to fifty times greater in molecular weight than the small molecule and is preferably less than about 60,000 Da; artisans will immediately appreciate that all the ranges and values within the explicitly stated ranges are contemplated. A more preferred range is a macromolecule that is about seven to about thirty times greater in molecular weight than the crosslinker and a most preferred range is about ten to twenty times difference in weight. Further, a macromolecular molecular weight of 5,000 to 50,000 is useful, as is a molecular weight of 7,000 to 40,000 or a molecular weight of 10,000 to 20,000. There are certain advantage to having a small molecule, such as diffusivity for completion of reactions.

Certain macromeric precursors are the crosslinkable, biodegradable, water-soluble macromers described in U.S. Patent No. 5,410,016 to Hubbell et al. These macromers are characterized by having at least two polymerizable groups, separated by at least one degradable region.

Synthetic precursors may be used. Synthetic refers to a molecule not found in nature or not normally found in a human. Some synthetic precursors are free of amino acids or free of amino acid sequences that occur in nature. Some synthetic precursors are polypeptides that are not found in nature or are not normally found in a human body, e.g., di-, tri-, or tetra-lysine. Some synthetic molecules have amino acid residues but only have one, two, or three that are contiguous, with the amino acids or clusters thereof being separated by non-natural polymers or groups. Polysaccharides or their derivatives are thus not synthetic.

Alternatively, natural proteins or polysaccharides may be adapted for use with these methods, e.g., collagens, fibrin(ogen)s, albumins, alginates, hyaluronic acid, and heparins. These natural molecules may further include chemical derivitization, e.g., synthetic polymer decorations. The natural molecule may be crosslinked via its native nucleophiles or after it is derivatized with functional groups, e.g., as in U.S. Patent Nos. 5,304,595, 5,324,775, 6,371,975, and 7,129,210. Natural refers to a molecule found in nature. Natural polymers, for example proteins or glycosaminoglycans, e.g., collagen, fibrinogen, albumin, and fibrin, may be crosslinked using reactive precursor species with electrophilic functional groups. Natural polymers normally found in the body are proteolytically degraded by proteases present in the body. Such polymers may be reacted via functional groups such as amines, thiols, or carboxyls on their amino acids or derivatized to have activatable functional groups. While natural polymers may be used in hydrogels, their time to gelation and ultimate mechanical properties must be controlled by appropriate introduction of additional functional groups and selection of suitable reaction conditions, e.g., pH.

Precursors may be made with a hydrophobic portion provided that the resultant hydrogel retains the requisite amount of water, e.g., at least about 20%. In some cases, the precursor is nonetheless soluble in water because it also has a hydrophilic portion. In other cases, the precursor makes dispersion in the water (a suspension) but is nonetheless reactable to from a crosslinked material. Some hydrophobic portions may include a plurality of alkyls, polypropylenes, alkyl chains, or other groups. Some precursors with hydrophobic portions are sold under the trade names PLURONIC F68, JEFFAMINE, or TECTRONIC. A hydrophobic molecule or a hydrophobic portion of a copolymer or the like is one that is sufficiently hydrophobic to cause the molecule (e.g., polymer or copolymer) to aggregate to form micelles or microphases involving the hydrophobic domains in an aqueous continuous phase or one that, when tested by itself, is sufficiently hydrophobic to precipitate from, or otherwise change phase while within, an aqueous solution of water at pH from about 7 to about 7.5 at temperatures from about 30 to about 50 degrees Centigrade.

Precursors may have, e.g., 2-100 arms, with each arm having a terminus, bearing in mind that some precursors may be dendrimers or other highly branched materials. An arm on a hydrogel precursor refers to a linear chain of chemical groups that connect a crosslinkable functional group to a polymer core. Some embodiments are precursors with between 3 and 300 arms; artisans will immediately appreciate that all the ranges and values within the explicitly stated ranges are contemplated, e.g., 4 to 16, 8 to 100, or at least 6 arms.

Thus hydrogels can be made, e.g., from a multi-armed precursor with a first set of functional groups and a low molecular-weight precursor having a second set of functional groups. For example, a six-armed or eight-armed precursor may have hydrophilic arms, e.g., polyethylene glycol, terminated with primary amines, with the molecular weight of the arms being about 1,000 to about 40,000; artisans will immediately appreciate that all ranges and values within the explicitly stated bounds are contemplated. Such precursors may be mixed with relatively smaller precursors, for example, molecules with a molecular weight of between about 100 and about 5000, or no more than about 800, 1000, 2000, or 5000 having at least about three functional groups, or between about 3 to about 16 functional groups; ordinary artisans will appreciate that all ranges and values between these explicitly articulated values are contemplated. Such small molecules may be polymers or non-polymers and natural or synthetic.

Precursors that are not dendrimers may be used. Dendritic molecules are highly branched radially symmetrical polymers in which the atoms are arranged in many arms and subarms radiating out from a central core. Dendrimers are characterized by their degree of structural perfection as based on the evaluation of both symmetry and polydispersity and require particular chemical processes to synthesize. Accordingly, an artisan can readily distinguish dendrimer precursors from non-dendrimer precursors. Dendrimers have a shape that is typically dependent on the solubility of its component polymers in a given environment, and can change substantially according to the solvent or solutes around it, e.g., changes in temperature, pH, or ion content.

Precursors may be dendrimers, e.g., as in U.S. Publication Nos. 2004/0086479 and 2004/0131582 and PCT Publication Nos. WO07005249, WO07001926 and WO06031358, or the U.S. counterparts thereof; dendrimers may also be useful as multifunctional precursors, e.g., as in U.S. Publication Nos. 2004/0131582 and 2004/0086479 and PCT Publication No. WO06031388. Dendrimers are highly ordered possess high surface area to volume ratios, and exhibit numerous end groups for potential functionalization. Embodiments include multifunctional precursors that are not dendrimers.

Some embodiments include a precursor that consists essentially of an oligopeptide sequence of no more than five residues, e.g., amino acids comprising at least one amine, thiol, carboxyl, or hydroxyl side chain. A residue is an amino acid, either as occurring in nature or derivatized thereof. The backbone of such an oligopeptide may be natural or synthetic. In some embodiments, peptides of two or more amino acids are combined with a synthetic backbone to make a precursor; certain embodiments of such precursors have a molecular weight in the range of about 100 to about 10,000 or about 300 to about 500 Artisans will immediately appreciate that all ranges and values between these explicitly articulated bounds are contemplated.

Precursors may be prepared to be free of amino acid sequences cleavable by enzymes present at the site of introduction, including free of sequences susceptible to attach by metalloproteinases and/or collagenases. Further, precursors may be made to be free of all amino acids, or free of amino acid sequences of more than about 50, 30, 20, 10, 9, 8, 7, 6, 5, 4, 3, 2, or 1 amino acids. Precursors may be non-proteins, meaning that they are not a naturally occurring protein and cannot be made by cleaving a naturally occurring protein and cannot be made by adding synthetic materials to a protein. Precursors may be non-collagen, non-fibrin, non-fibrinogen, and non-albumin, meaning that they are not one of these proteins and are not chemical derivatives of one of these proteins. The use of non-protein precursors and limited use of amino acid sequences can be helpful for avoiding immune reactions, avoiding unwanted cell recognition, and avoiding the hazards associated with using proteins derived from natural sources. Precursors can also be non-saccharides (free of saccharides) or essentially non-saccharides (free of more than about 5% saccharides by w/w of the precursor molecular weight. Thus a precursor may, for example, exclude hyaluronic acid, heparin, or gellan. Precursors can also be both non-proteins and non-saccharides. The term protein, as used herein, is a broad term referring to a polypeptide; the term protein fragment may be used to refer to a less than complete sequence of a wild-type protein: precursors or therapeutic agents may be protein fragments.

Peptides may be used as precursors. In general, peptides with less than about 10 residues are preferred, although larger sequences (e.g., proteins) may be used. Artisans will immediately appreciate that every range and value within these explicit bounds is included, e.g., 1-10, 2-9, 3-10, 1, 2, 3, 4, 5, 6, or 7. Some amino acids have nucleophilic groups (e.g., primary amines or thiols) or groups that can be derivatized as needed to incorporate nucleophilic groups or electrophilic groups (e.g., carboxyls or hydroxyls). Polyamino acid polymers generated synthetically are normally considered to be synthetic if they are not found in nature and are engineered not to be identical to naturally occurring biomolecules.

Some organogels and hydrogels are made with a polyethylene glycol-containing precursor. Polyethylene glycol (PEG, also referred to as polyethylene oxide when occurring in a high molecular weight) refers to a polymer with a repeat group (CH₂CH₂O)ₙ, with n being at least 3. A polymeric precursor having a polyethylene glycol thus has at least three of these repeat groups connected to each other in a linear series. The polyethylene glycol content of a polymer or arm is calculated by adding up all of the polyethylene glycol groups on the polymer or arm, even if they are interrupted by other groups. Thus, an arm having at least 1000 MW polyethylene glycol has enough CH₂CH₂O groups to total at least 1000 MW. As is customary terminology in these arts, a polyethylene glycol polymer does not necessarily refer to a molecule that terminates in a hydroxyl group. Molecular weights are abbreviated in thousands using the symbol k, e.g., with 15K meaning 15,000 molecular weight, i.e., 15,000 Daltons. NH2 refers to an amine termination. SG refers to succinimidyl glutarate. SS refers to succinimidyl succinate. SAP refers to succinimidyl adipate. SAZ refers to succinimidyl azelate. SS, SG, SAP and SAZ are succinimidyl esters that have an ester group that degrades by hydrolysis in water. Hydrolytically degradable or water-degradable thus refers to a material that would spontaneously degrade *in vitro* in an excess of water without any enzymes or cells present to mediate the degradation. A time for degradation refers to effective disappearance of the material as judged by the naked eye. Trilysine (also abbreviated LLL) is a synthetic tripeptide. PEG and/or hydrogels, as well as compositions that comprise the same, may be provided in a form that is pharmaceutically acceptable, meaning that it is highly purified and free of contaminants, e.g., pyrogens.

### Hydrogel Structures

The hydrogel's structure and the material composition of the hydrogel's precursors determine its properties. Precursor factors include properties such as biocompatibility, water solubility, hydrophilicity, molecular weight, arm length, number of arms, functional groups, distance between crosslinks, degradability, and the like. The choice of reaction conditions also effects the hydrogel's structure and properties, including choices of solvents, reaction schemes, reactant concentrations, solids content, and the like. There can be a variety of ways to achieve certain properties, or combination of properties. On the other hand some properties are in tension with each other, for instance brittleness may increase as a distance between crosslinks or solids content increases. Strength may be increased by increasing the number of crosslinks but swelling may thereby be reduced. Artisans will appreciate that the same materials may be used to make matrices with a great range of structures that will have highly distinct mechanical properties and performance, such that the achievement of a particular property should not be merely assumed based on the general types of precursors that are involved.

The spacing between molecular strands of the hydrogel (the matrix) affects several hydrogel properties, including a rate of diffusion of molecules. The crosslinking density can be controlled by the choice of the overall molecular weight of the precursor(s) used as crosslinker(s) and other precursor(s) and the number of functional groups available per precursor molecule. A lower molecular weight between crosslinks such as 200 will give much higher crosslinking density as compared to a higher molecular weight between crosslinks such as 500,000; artisans will immediately appreciate that all ranges and values within this range are contemplated and supported, e.g., 200 to 250,000, 500 to 400,000, and so forth. The crosslinking density also may be controlled by the overall percent solids of the crosslinker and functional polymer solutions. Yet another method to control crosslink density is by adjusting the stoichiometry of nucleophilic functional groups to electrophilic functional groups. A one to one ratio leads to the highest crosslink density. Precursors with longer distances between crosslinkable sites form gels that are generally softer, more compliant, and more elastic. Thus an increased length of a water-soluble segment, such as a polyethylene glycol, tends to enhance elasticity to produce desirable physical properties. Thus certain embodiments are directed to precursors with water soluble segments having molecular weights in the range of 2,000 to 100,000; artisans will immediately appreciate that all the ranges and values within the explicitly stated ranges are contemplated, e.g., 5,000 to 35,000. Thus embodiments include materials (organogels, hydrogels, xerogels, a (first) material that is placed within an envelope or coating of a second material, or the (second) material used for an envelope) with a molecular weight between crosslinks of at least 2000, at least 4000, or from 2000-250,000; Artisans will immediately appreciate that all ranges and values between the explicitly stated bounds are contemplated, with, e.g., any of the following being available as an upper or lower limit: 3000, 5000, 10,000, 50,000, 100,000. The solids content of the hydrogel (or the xerogel or organogel that gives rise to a hydrogel) can affect its mechanical properties and biocompatibility and reflects a balance between competing requirements. A relatively low solids content is useful, e.g., between about 2.5% to about 20%, artisans will immediately appreciate that this range is including all ranges and values there between, e.g., about 2.5% to about 10%, about 5% to about 15%, or less than about 15%. Solids content and distance between crosslinks is measured at the equilibrium water content of the material in water. Thus embodiments include materials (organogels, hydrogels, xerogels, a (first) material that is placed within an envelope or coating of a second material, or the (second) material used for an envelope) with a solids content from about 2.5% to about 20%, Artisans will immediately appreciate that all ranges and values between the explicitly stated bounds are contemplated. Solids content percentages are w/w measured at equilibrium water content.

One way to construct the materials so that the delay is controlled or minimized is to design the hydrogels with different rates of diffusion for the agent. Often the molecular weight (MW) of the agent is a controlling variable. There are a number of approaches for relating hydrogel properties to diffusion. These include the free volume theory, the hydrodynamic theory, the obstruction theory, combination theories, and parameters such as mesh size, sieving terms, distributions of openings between chains, and so forth (Amsden, Macromolecules (1998) 31:8382-8395). In practice, however, hydrogels can be made with various distances between their crosslinks and tested for a particular molecule to create a hydrogel that provides a desired diffusion rate. In general, a distance between crosslinks that is large compared to the molecule's size provides for a high rate of diffusion, a distance between crosslinks that is small compared to the molecule's size provides for a slow diffusion, and a distance between crosslinks that is smaller than the molecule provides for essentially no diffusion. A molecule's molecular weight is generally a useful measure of it size. There are other factors that can be important and these can be accounted for when creating the hydrogel: for instance, interactions between the molecule and the hydrogel, such as affinity or charge-charge, and solvent effects such as hydrophobicity of the molecule.

Accordingly, embodiments include a biomedical sustained release system for use in a patient comprising a collection of particles that comprise a first biodegradable material that is a hydrogel or a xerogel and a therapeutic agent with the first material, before biodegradation, having a first rate of release for the therapeutic agent as measured in physiological solution, and a second material that is a hydrogel or xerogel that (before biodegradation) delays release by a predetermined amount and, optionally, is free of the therapeutic agent until such time as the agent diffuses from the particles into the second hydrogel. The predetermined amount of release can be described with reference to a controlled release profile as already described, e.g., as in Fig. 5. The rate of release from a hydrogel is measured in vitro in a great excess of physiological solution, enough so that the solution is very large relative to the hydrogel so that the agent does not accumulate in the solution and reduce the effective rate of release. The solution, for testing, is phosphate buffer solution at pH 7.4 osmotically balanced for physiological conditions, as is customary in these arts. Also, pH 7.2 is often used for specifically simulating the ocular tissue environment.

Various therapeutic agents are described herein; they may be incorporated into these systems. Their sizes are well known or easily determined. Their release rates can be readily established. The agent can be one as set forth specifically herein or can be an agent having a molecular weight of less than about 450 kDa or in a range from 200 Da to about 450kDa; artisans will immediately appreciate that all ranges and values within this range are contemplated and supported, e.g., about 500 Da to about 250kDa, about 10kDa to about 180kDa, no more than about 205kDa, about 100kDa to about 255kDa, and so forth. Release rates reflect the condition of the system at the time of implantation. The systems can be biodegradable and the relative rates may change over time as degradation takes place. Since the release rate through the second material is very high, however, it is permissive to passage of the molecule and not essential to the control of the delivery process. The particles inside the envelope of hydrogel and the condition of the agents in the particles are controlling for release of the agent. The envelope can affect the rate of release but only incidentally.

In fact, the second material, which, in vivo, is a hydrogel that at least partially coats the first hydrogel, has a role in biocompatibility. It was observed that the hydrogel particles, when loaded with agents, specifically proteins not native to the animal model of the in vivo test, elicited some unwanted biological effects that indicated a lack of biocompatibility. But the same materials, when coated with a hydrogel, were more biocompatible. Rabbit eyes, which are a highly sensitive model, were used to establish these effects. Without being bound to a particular theory, it is theorized that macrophages or other immune system cells were more responsive to particles than the monolithic coating of a second material. The particles have a higher surface area and also have more resemblance to cell, virus, or tissue surfaces as compared to the sheet-like coating. Further, or alternatively, the particles contained the agents and might not have coated all of the agent molecules in their entirety, so that the immune system cells could interact with them before the hydrogel degraded. The outer enveloping hydrogel is sized to keep all cells out but to allow the agents from the particles to rapidly and freely diffuse. The term encapsulating, when used, refers to placing a coating over all of the particles that are injected or otherwise placed into a patient. As is evident, embodiments include choosing the second material to have, relative to the first material (such as particles), a lower value for one or more of: molecular weight, solids content, distance between crosslinks, and persistence in vivo. The first material (hydrogel etc.) may be in particulate form, and be a collection of particles with a particular size range or distribution as described elsewhere herein. Particles are useful for drug delivery, however, other objects may be coated, e.g., medical implants, implantable materials, rods, rods with a dimension of at least 1 mm, punctal plugs, etc.

Example 1 shows a comparison of an in situ formed hydrogel coating on release kinetics of a therapeutic agent. In this study, fast degrading hydrogel particles were used; these conveniently provide a high rate of therapeutic agent release so that the effects of encapsulating the particles in a hydrogel coating intended to be permissive to passage of the agent was tested. A small but manageable difference in release rate was observed, with both formulations releasing fully in less than one week. This indicates that, for a particulate hydrogel-based protein delivery system designed for slow release, a relatively high-release hydrogel can be overlayed to make a combined system. In Example 2, it was observed that the encapsulating hydrogel of Example 1 had an important effect on improving biocompatibility. Hydrogel particles coated with an encapsulating hydrogel showed a markedly lower inflammation as compared to OTX-14 around the retina (Table 2).

### Functional Groups

The precursors for covalent crosslinking have functional groups that react with each other to form the material via covalent bonds, either outside a patient, or *in situ.* The functional groups generally are polymerizable, a broad category that encompasses free radical, addition, and condensation polymerization and also groups for electrophile-nucleophile reactions. Various aspects of polymerization reactions are discussed in the precursors section herein.

Thus in some embodiments, precursors have a polymerizable group that is activated by photoinitiation or redox systems as used in the polymerization arts, or electrophilic functional groups, for instance: carbodiimidazole, sulfonyl chloride, chlorocarbonates, n-hydroxysuccinimidyl ester, succinimidyl ester or sulfasuccinimidyl esters, or as in U.S. Patent Nos. 5,410,016 or 6,149,931. The nucleophilic functional groups may be, for example, amine, hydroxyl, carboxyl, and thiol. Another class of electrophiles are acyls, e.g., as in U.S. Patent No. 6,958,212, which describes, among other things, Michael addition schemes for reacting polymers.

Certain functional groups, such as alcohols or carboxylic acids, do not normally react with other functional groups, such as amines, under physiological conditions (e.g., pH 7.2-11.0, 37°C). However, such functional groups can be made more reactive by using an activating group such as N-hydroxysuccinimide. Certain activating groups include carbonyldiimidazole, sulfonyl chloride, aryl halides, sulfosuccinimidyl esters, N-hydroxysuccinimidyl ester, succinimidyl ester, epoxide, aldehyde, maleimides, imidoesters and the like. The N-hydroxysuccinimide esters or N-hydroxysulfosuccinimide (NHS) groups are useful groups for crosslinking of proteins or amine-containing polymers, e.g., amino terminated polyethylene glycol. An advantage of an NHS-amine reaction is that the reaction kinetics are favorable, but the gelation rate may be adjusted through pH or concentration. The NHS-amine crosslinking reaction leads to formation of N-hydroxysuccinimide as a side product. Sulfonated or ethoxylated forms of N-hydroxysuccinimide have a relatively increased solubility in water and hence their rapid clearance from the body. An NHS-amine crosslinking reaction may be carried out in aqueous solutions and in the presence of buffers, e.g., phosphate buffer (pH 5.0-7.5), triethanolamine buffer (pH 7.5-9.0), or borate buffer (pH 9.0-12), or sodium bicarbonate buffer (pH 9.0-10.0). Aqueous solutions of NHS based crosslinkers and functional polymers preferably are made just before the crosslinking reaction due to reaction of NHS groups with water. The reaction rate of these groups may be delayed by keeping these solutions at lower pH (pH 4-7). Buffers may also be included in the hydrogels introduced into a body.

In some embodiments, each precursor comprises only nucleophilic or only electrophilic functional groups, so long as both nucleophilic and electrophilic precursors are used in the crosslinking reaction. Thus, for example, if a crosslinker has nucleophilic functional groups such as amines, the functional polymer may have electrophilic functional groups such as N-hydroxysuccinimides. On the other hand, if a crosslinker has electrophilic functional groups such as sulfosuccinimides, then the functional polymer may have nucleophilic functional groups such as amines or thiols. Thus, functional polymers such as proteins, poly(allyl amine), or amine-terminated di-or multifunctional poly(ethylene glycol) can be used.

One embodiment has reactive precursor species with 2 to 16 nucleophilic functional groups each and reactive precursor species with 2 to 16 electrophilic functional groups each; artisans will immediately appreciate that all the ranges and values within the explicitly stated ranges are contemplated, for instance 2, 3, 4, 5, 6, 7, 8, 9, 10, 11, 12, 13, 14, 15, or 16 groups.

The functional groups may be, e.g., electrophiles reactable with nucleophiles, groups reactable with specific nucleophiles, e.g., primary amines, groups that form amide bonds with materials in the biological fluids, groups that form amide bonds with carboxyls, activated-acid functional groups, or a combination of the same. The functional groups may be, e.g., a strong electrophilic functional group, meaning an electrophilic functional group that effectively forms a covalent bond with a primary amine in aqueous solution at pH 9.0 at room temperature and pressure and/or an electrophilic group that reacts by a of Michael-type reaction. The strong electrophile may be of a type that does not participate in a Michaels-type reaction or of a type that participates in a Michaels-type reaction.

A Michael-type reaction refers to the 1, 4 addition reaction of a nucleophile on a conjugate unsaturated system. The addition mechanism could be purely polar, or proceed through a radical-like intermediate state(s); Lewis acids or appropriately designed hydrogen bonding species can act as catalysts. The term conjugation can refer both to alternation of carbon-carbon, carbon-heteroatom or heteroatom-heteroatom multiple bonds with single bonds, or to the linking of a functional group to a macromolecule, such as a synthetic polymer or a protein. Michael-type reactions are discussed in detail in U.S. Patent No. 6,958,212, which is hereby incorporated by reference herein in its entirety for all purposes to the extent it does not contradict what is explicitly disclosed herein.

Examples of strong electrophiles that do not participate in a Michaels-type reaction are: succinimides, succinimidyl esters, or NHS-esters. Examples of Michael-type electrophiles are acrylates, methacrylates, methylmethacrylates, and other unsaturated polymerizable groups.

### Initiating Systems

Some precursors react using initiators. An initiator group is a chemical group capable of initiating a free radical polymerization reaction. For instance, it may be present as a separate component, or as a pendent group on a precursor. Initiator groups include thermal initiators, photoactivatable initiators, and oxidation-reduction (redox) systems. Long wave UV and visible light photoactivatable initiators include, for example, ethyl eosin groups, 2, 2-dimethoxy-2-phenyl acetophenone groups, other acetophenone derivatives, thioxanthone groups, benzophenone groups, and camphorquinone groups. Examples of thermally reactive initiators include 4, 4' azobis (4-cyanopentanoic acid) groups, and analogs of benzoyl peroxide groups. Several commercially available low temperature free radical initiators, such as V-044, available from Wako Chemicals USA, Inc., Richmond, Va., may be used to initiate free radical crosslinking reactions at body temperatures to form hydrogel coatings with the aforementioned monomers.

Metal ions may be used either as an oxidizer or a reductant in redox initiating systems. For example, ferrous ions may be used in combination with a peroxide or hydroperoxide to initiate polymerization, or as parts of a polymerization system. In this case, the ferrous ions would serve as a reductant. Alternatively, metal ions may serve as an oxidant. For example, the ceric ion (4+ valence state of cerium) interacts with various organic groups, including carboxylic acids and urethanes, to remove an electron to the metal ion, and leave an initiating radical behind on the organic group. In such a system, the metal ion acts as an oxidizer. Potentially suitable metal ions for either role are any of the transition metal ions, lanthanides and actinides, which have at least two readily accessible oxidation states. Particularly useful metal ions have at least two states separated by only one difference in charge. Of these, the most commonly used are ferric/ferrous; cupric/cuprous; ceric/cerous; cobaltic/cobaltous; vanadate V vs. IV; permanganate; and manganic/manganous. Peroxygen containing compounds, such as peroxides and hydroperoxides, including hydrogen peroxide, t-butyl hydroperoxide, t-butyl peroxide, benzoyl peroxide, cumyl peroxide may be used.

An example of an initiating system is the combination of a peroxygen compound in one solution, and a reactive ion, such as a transition metal, in another. In this case, no external initiators of polymerization are needed and polymerization proceeds spontaneously and without application of external energy or use of an external energy source when two complementary reactive functional groups containing moieties interact at the application site.

### Precursors as coatings

Embodiments include a medical device having at least a partial coating of precursors that form a hydrogel in situ upon exposure to aqueous solution. The term medical device is broad and encompasses drug delivery devices, drug depots for delivery of a drug, an intraocular drug depot, an implantable, a prosthesis, and objects made to contact a physiological fluid. An example is a punctal plug, an intraocular drug depot, or a fiber used for a medical device, wherein the plug or the fiber is completely or partially coated with the precursors. A method of applying a coating comprises dipping the device or the portion to be coated into a melt of polymer or polymers (precursors) that form the coating. Polymers that melt at a temperature of no more than about, e.g., 100 degrees C are melted, in the absence of solvents. The plug, or portion thereof, is dipped into the melt. The melt is allowed to cool to a solid, and remains a solid at 37 degrees C. Instead of dipping the plug into the melt, the melts may be otherwise applied, e.g., brushing, rolling, dropping melt onto the plug, and so forth. The term melt, in the context of a polymer, refers to a polymer that is in a liquid state but is not dissolved in a solvent, or the polymer acts as its own solvent. Some other materials may be present in the melt, but they are not solvents for the melt. It is recognized that some small amount of a solvent can be present in a concentration that is not effective to dissolve a substantial portion of the polymers in the melt, e.g., no more than 10%, weight per total weight; Artisans will immediately appreciate that all ranges and values between the explicitly stated bounds are contemplated, with any of the following being available as an upper or lower limit: 0.1, 0.2., 0.5, 1, 2, 3, 4, 5, 6, 7, 8, 9, or 10, referring to % weight/total weight. Agents may be present in the melt that assist in adjusting its melting point. For instance, addition of agents that reduce the forces of association between polymers may be added to reduce a melting point; such agents may be non-solvents or solvent. Such agents may be added at, e.g., no more than 10%, weight per total weight; Artisans will immediately appreciate that all ranges and values between the explicitly stated bounds are contemplated, with any of the following being available as an upper or lower limit: 0.1, 0.2., 0.5, 1, 2, 3, 4, 5, 6, 7, 8, 9, or 10, referring to % weight/total weight. Also, the use of branched polymers may be used to adjust melting temperatures.

An example of polymers that melt at a temperature that is reasonable for dipping the punctal plug or other device without damage includes PEGs, with the melting point being related to the MW of the PEG. A PEG of about 8,000 MW has been tested and is useful. Other MWs for PEGs are, for instance, from about 2,000 to about 100,000 (MWs for polymers refer to a weight average molecular weight unless otherwise specified). In general, the polymer or mixture of polymers is chosen to set the desired melt temperature and the target dissolving time.

A method of applying a precursor coating to a punctal plug or other device comprises exposing a punctal plug or other device to a solution comprising the polymer(s) that will form the coating, with the polymer(s) being in solution in a solvent that is not a solvent for the punctal plug. The solvent, in general, is nonaqueos and is an organic solvent. Examples of organic solvents are dimethlycarbonate, dimethylformamide dimethyl sulfoxide, n-methyl pyrrolidinone, dimethyl sulfoxide, ethyl lactate, N-dicyclohexylcarbodiimide. Other solvents that may be used are alcohols: ethanol, isopropanol, 1, 2-propane diol, 1, 4-butane diol.

The precursor coatings may be made using water to dissolve coating materials to make a solution that is sprayed onto a plug to make a water soluble coating, a process referred to as a fluidized bed. An alternative configuration could use a coating material that dissolves in a non-aqueous solvent to form a non-aqueous solution.

The precursor coating does not have to be a melt. The precursors may be disposed in a suitable solvent and applied to the plug or other device, or applied in a dry form. The coatings may comprise excipients, e.g., binding agents, nonreactive materials or nonreactive polymers, plasticizers, buffer agents, visualization agents, dyes, or salts.

### Visualization agents

A visualization agent may be used as a powder in a xerogel/hydrogel; it reflects or emits light at a wavelength detectable to a human eye so that a user applying the hydrogel could observe the object when it contains an effective amount of the agent. Agents that require a machine aid for imaging are referred to as imaging agents herein, and examples include: radioopaque contrast agents and ultrasound contrast agents. Some biocompatible visualization agents are FD&C BLUE #1, FD&C BLUE #2, and methylene blue. These agents are preferably present in the final electrophilic-nucleophilic reactive precursor species mix at a concentration of more than 0.05 mg/ml and preferably in a concentration range of at least 0.1 to about 12 mg/ml, and more preferably in the range of 0.1 to 4.0 mg/ml, although greater concentrations may potentially be used, up to the limit of solubility of the visualization agent. Visualization agents may be covalently linked to the molecular network of the xerogel/hydrogel, thus preserving visualization after application to a patient until the hydrogel hydrolyzes to dissolution. Visualization agents may be selected from among any of the various non-toxic colored substances suitable for use in medical implantable medical devices, such as FD&C BLUE dyes 3 and 6, eosin, methylene blue, indocyanine green, or colored dyes normally found in synthetic surgical sutures. Reactive visualization agents such as NHS-fluorescein can be used to incorporate the visualization agent into the molecular network of the xerogel/hydrogel. The visualization agent may be present with either reactive precursor species, e.g., a crosslinker or functional polymer solution. The preferred colored substance may or may not become chemically bound to the hydrogel.

### Biodegradation

An organogel and/or xerogel and/or hydrogel may be formed so that, upon hydration in physiological solution, a hydrogel is formed that is water-degradable, as measurable by the hydrogel losing its mechanical strength and eventually dissipating *in vitro* in an excess of water by hydrolytic degradation of water-degradable groups. This test is predictive of hydrolytically-driven dissolution *in vivo,* a process that is in contrast to cell or protease-driven degradation. Significantly, however, polyanhydrides or other conventionally-used degradable materials that degrade to acidic components tend to cause inflammation in tissues. The hydrogels, however, may exclude such materials, and may be free of polyanhydrides, anhydride bonds, or precursors that degrade into acid or diacids. The term degradation by solvation in water, also referred to as dissolving in water, refers to a process of a matrix gradually going into solution in, which is a process that cannot take place for a covalently crosslinked material and materials insoluble in water.

For example, electrophilic groups such as SG (N-hydroxysuccinimidyl glutarate), SS (N-hydroxysuccinimidyl succinate), SC (N-hydroxysuccinimidyl carbonate), SAP (N-hydroxysuccinimidyl adipate) or SAZ (N-hydroxysuccinimidyl azelate) may be used and have esteric linkages that are hydrolytically labile. More linear hydrophobic linkages such as pimelate, suberate, azelate or sebacate linkages may also be used, with these linkages being less degradable than succinate, glutarate or adipate linkages. Branched, cyclic or other hydrophobic linkages may also be used. Polyethylene glycols and other precursors may be prepared with these groups. The crosslinked hydrogel degradation may proceed by the water-driven hydrolysis of the biodegradable segment when water-degradable materials are used. Polymers that include ester linkages may also be included to provide a desired degradation rate, with groups being added or subtracted near the esters to increase or decrease the rate of degradation. Thus it is possible to construct a hydrogel with a desired degradation profile, from a few days to many months, using a degradable segment. If polyglycolate is used as the biodegradable segment, for instance, a crosslinked polymer could be made to degrade in about 1 to about 30 days depending on the crosslinking density of the network. Similarly, a polycaprolactone based crosslinked network can be made to degrade in about 1 to about 8 months. The degradation time generally varies according to the type of degradable segment used, in the following order: polyglycolate < polylactate < polytrimethylene carbonate < polycaprolactone. Thus it is possible to construct a hydrogel with a desired degradation profile, from a few days to many months, using a degradable segment. Some embodiments include precursors that are free of adjacent ester groups and/or have no more than one ester group per arm on one or more of the precursors: control of the number and position of the esters can assist in uniform degradation of the hydrogel.

A biodegradable linkage in the organogel and/or xerogel and/or hydrogel and/or precursor may be water-degradable or enzymatically degradable. Illustrative water-degradable biodegradable linkages include polymers, copolymers and oligomers of glycolide, dl-lactide, 1-lactide, dioxanone, esters, carbonates, and trimethylene carbonate. Illustrative enzymatically biodegradable linkages include peptidic linkages cleavable by metalloproteinases and collagenases. Examples of biodegradable linkages include polymers and copolymers of poly(hydroxy acid)s, poly(orthocarbonate)s, poly(anhydride)s, poly(lactone)s, poly(aminoacid)s, poly(carbonate)s, and poly(phosphonate)s.

If it is desired that a biocompatible crosslinked matrix be biodegradable or absorbable, one or more precursors having biodegradable linkages (or just one biodegradable linkage, for example an ester) present in between the functional groups may be used. The biodegradable linkage optionally also may serve as the water soluble core of one or more of the precursors used to make the matrix. For each approach, biodegradable linkages may be chosen such that the resulting biodegradable biocompatible crosslinked polymer will degrade or be absorbed in a desired period of time.

### Hydrogel/Xerogel/Organogel Loading with Agents; Preparation as Particles

One approach for making a hydrogel or organogel with a therapeutic agent is to form it around the agent. For instance, a first precursor is added to a solvent-protein mixture, followed by a second precursor that is reactive with the first precursor to form crosslinks. After formation of the matrix in the solvent, the solvent may be removed to form a xerogel. Potential processes include, e.g., precipitation with non-solvent, nitrogen sweep drying, vacuum drying, freeze-drying, a combination of heat and vacuum, and lyophilization. If molten precursors are used in the absence of a tertiary solvent, there is no need to employ any solvent removal process. Upon cooling the material forms a rubbery solid (if above Tg), a semirigid semicrystalline material (if below Tm and above Tg) or a rigid glassy solid (if below Tg). These materials are more dense than xerogels formed from organic solvents. When filled with particles of other materials, e.g., therapeutic agents, buffer salts, visualization agents, they can be highly porous, since the solid particles create and fill the pores.

In some embodiments, the agent or agents are present in a separate phase when precursors are reacted. The separate phase could be oil (oil-in water emulsion), or an immiscible solvent, a liposome, a micelle, a biodegradable vehicle, and the like. Biodegradable vehicles in which the active agent may be present include: encapsulation vehicles, such as microparticles, microspheres, microbeads, micropellets, where the active agent is encapsulated in a bioerodable or biodegradable polymers such as polymers and copolymers of: poly(anhydride), poly(hydroxy acid)s, poly(lactone)s, poly(trimethylene carbonate), poly(glycolic acid), poly(lactic acid), poly(glycolic acid)-co-poly(glycolic acid), poly(orthocarbonate), poly(caprolactone), crosslinked biodegradable hydrogel networks like fibrin glue or fibrin sealant, caging and entrapping molecules, like cyclodextrin, molecular sieves and the like. Microspheres made from polymers and copolymers of poly(lactone)s and poly(hydroxy acid) are particularly preferred as biodegradable encapsulation vehicles. The therapeutic agent or encapsulated therapeutic agent may be present in solution or suspended form. Some agents are highly soluble while others are effectively insoluble in aqueous solution and can form their own phase when exposed to aqueous solvent.

Therapeutic agents can be in solid particulate form within the hydrogel/organogel/xerogel, e.g., as a powder. For instance, water soluble biologics (e.g., proteins) in solid phase can be ground or otherwise formed into a fine powder that is added to the precursors when a matrix is formed. The protein or other water soluble biologic in the xerogel may all be in a solid phase, may be all crystalline, partially crystalline, or essentially free of crystals (meaning more than 90% free of crystals w/w; artisans will immediately appreciate that all the ranges and values within the explicitly stated ranges are contemplated). A powder of a protein refers to a powder made from one or more proteins. Similarly, powders of water soluble biologics are powders having particles made of one or more water soluble biologics. The powders and/or xerogels and/or organogels and/or hydrogels that contain them may be free of encapsulating materials and be free of one or more of a liposome, micelle, or nanocapsule. Further, a protein particle or a water soluble biologic particle may be made that is free of one or more of: binders, non-peptidic polymers, surfactants, oils, fats, waxes, hydrophobic polymers, polymers comprising alkyl chains longer than 4 CH2 groups, phospholipids, micelle-forming polymers, micelle-forming compositions, amphiphiles, polysaccharides, polysaccharides of three or more sugars, fatty acids, and lipids. Lyophilized, spray dried or otherwise processed proteins are often formulated with sugars such as trehalose to stabilize the protein through the lyophilization or other processes used to prepare the proteins. These sugars may be allowed to persist in the particle throughout the organogel/xerogel process. The particles may be made to comprise between about 20% and about 100% (dry w/w) protein; artisans will immediately appreciate that all the ranges and values within the explicitly stated ranges are contemplated, e.g., about 50% to about 80% or at least 90% or at least about 99%. A number of factors can be controlled that contribute to processing and delivery of a protein without denaturation. The protein may be prepared as a powder, with the powder particle size being chosen in light of the size of the ultimate hydrogel/organogel/xerogel particle. Organic solvents for the proteins may be chosen so that the proteins are not solvated by the organic solvents and are compatible with the protein. Another factor is oxygen, and elimination of oxygen is helpful in processing to avoid denaturation. Another factor is chemical reactions. These may be avoided by keeping the protein in a solid phase and free of solvents that dissolve the protein until such time as the protein is implanted.

An organogel or hydrogel may be formed and then reduced to particles that are subsequently treated to remove the organic or aqueous solvent or solvents to form a xerogel. For an injectable form, the organogel or hydrogel can be macerated, homogenized, extruded, screened, chopped, diced, or otherwise reduced to a particulate form. Alternatively, the organogel or hydrogel can be formed as a droplet or a molded article containing the suspended protein particles. One process for making such particles involves creation of a material that is broken up to make the particles. One technique involves preparing the organogel or hydrogel with protein particles and grinding it, e.g., in a ball mill or with a mortar and pestle. The matrix may be chopped or diced with knives or wires. Or the matrix may be cut-up in a blender or homogenizer. Another process involves forcing the organogel through a mesh, collecting the fragments, and passing them through the same mesh or another mesh until a desired size is reached.

The particles of biologics or the particles of organogels or the particles of the xerogels may be separated into collections with a desired size range and distribution of sizes by a variety of methods. Very fine control of sizing is available, with sizes ranging from less than 1 micron to several mm, and with a mean and range of particles sizes being controllable with a narrow distribution. Artisans will immediately appreciate that all the ranges and values within the explicitly stated ranges are contemplated, e.g., from about 0.1 to about 10 µm or from about 1 to about 30 µm. About 1 to about 500 microns is another such range that is useful, with sizes falling throughout the range and having a mean sizing at one value within the range, and a standard deviation centered around the mean value, e.g., from about 1% to about 100%. A simple method for sizing particles involves using custom-made or standardized sieve mesh sizes. Another method to measure particle size is with a laser diffraction particle size analyzer, such as the Coulter LS 200, which analyzes particles when suspended in a liquid such as saline. The term particle is broad and includes spheres, discs, and irregularly shaped particles. A spheroidal particle refers to a particle wherein the longest central axis (a straight line passing through the particle's geometric center) is no more than about twice the length of other central axes, with the particle being a literally spherical or having an irregular shape. A rod-shaped particle refers to a particle with a longitudinal central axis more than about twice the length of the shortest central axis. Embodiments include making a plurality of collections of particles, with the collections having different rates of degradation *in vivo,* and mixing collections to make a biomaterial having a degradation performance as desired.

Particles may be prepared as collections having a certain average volume, average mean volume, or distribution of sizes that falls within a certain range of volumes (meaning at least 95% w/w of the particles are distributed within the range). An embodiment is a collection of particles having one or more of: an average volume, an average mean volume, or a distribution of sizes from about 0.02 µm³ to about 2 mm³; artisans will immediately appreciate that all ranges and values within this range are contemplated and supported, e.g., from 0.025 µm³ to 1 mm3, 0.03 µm³ to 1.5 mm3, and so forth. Further, the total volume of the collection of particles and/or the total volume of all of the hydrogels in the systems for human injection may have a value from about 0.005 to about 2.5 milliliters (ml); artisans will immediately appreciate that all ranges and values within this range are contemplated and supported, e.g., 0.005 to 1 ml, 0.1 ml to 1.5 ml, and so forth. The particles may have a diameter (referring to a longest dimension if not symmetrical) from 0.01 microns to 2 mm; Artisans will immediately appreciate that all ranges and values between the explicitly stated bounds are contemplated, with, e.g., any of the following being available as an upper or lower limit: 1, 5, 10, 20, 50, or 100 nanometers, 0.1, 0.2, 0.5, 1, 10, 20, 30 , 40, 50, 100, 200, 300, 500, 1000 microns, 1, 1.5, or 2 mm. In particular tissues, such as in the various structures of the eye that have been targeted for drug delivery, the volume available for occupation by a drug delivery depot is limited. For example, the suprachoroidal space could contain a depot of up to about 100µl, or 200 µl if it is conformal to the shape of the space. Likewise, up to 100µl, or 200 µl can be injected into the vitreous humor as long as the depot does not infringe on the visual axis. Other sites, for instance subconjunctival delivery, could accommodate larger depots, since the tissue can expand to accommodate.

Alternatively, instead of particles, the hydrogel/organogel/xerogels may be formed as, or as part of, a medical device or medical implant. A device may be used in or on the body. An implant is at least partially implanted inside the body, or may be implanted entirely within the body. Examples of devices are punctal plugs, objects with a rod shape, drug delivery devices, patches, and drug depots that are intravascular or extravascular but in contact with at least a portion of a blood vessel or associated structure (e.g., adventitia). The hydrogel/organogel/xerogels may be formed ex vivo for use with the device, e.g., formed and lyophilized to make a xerogel, or formed in situ, e.g., by providing at least a partial coating of precursors that form a hydrogel in vivo upon exposure to aqueous solution.

### Administration

An embodiment is a hydrogel formed by *in situ* polymerization around hydrogel and/or xerogel particles, with the particles containing a therapeutic agent. The *in situ* formed hydrogel envelopes the particles and may encapsulate them. When the particles are well mixed with the enveloping hydrogel, they will all take-on a coating of the same and will thus be encapsulated. In other cases the particles are placed in the patent and then the hydrogel is applied with the result that there may be only a partial coating of the particles since.

In use, the hydrogel particles are mixed with precursors and injected into the site of intended use in the patient. The precursors react with each other to form the enveloping hydrogel. The particles can be made with a first diffusivity for an agent and the enveloping hydrogel can be made with a second diffusivity. A needle, cannula, trocar, sprayer, or other applicator may be used. Administration of the hydrogels and/or xerogels may also involve hydration in advance, at about the time of use, or at the point of use. Or xerogels may be implanted without hydration and allowed to hydrate *in situ.*

The materials described herein may be used to deliver drugs or other therapeutic agents (e.g., imaging agents or markers). One mode of application is to apply a mixture of xerogel/hydrogel particles and other materials (e.g., therapeutic agent, buffer, accelerator, initiator) through a needle, microneedle, cannula, catheter, or hollow wire to a site. The mixture may be delivered, for instance, using a manually controlled syringe or mechanically controlled syringe, e.g., a syringe pump. Alternatively, a dual syringe or multiple-barreled syringe or multi-lumen system may be used to mix the xerogel/hydrogel particles at or near the site with a hydrating fluid and/or other agents. Some sites require a careful administration process, e.g., in an eye. Fine needles may be used and/or needles with a limited length. The work may be performed, if helpful, under magnification, with a stereoscope, with guided imaging, or with robots (for instance as described by Eindhoven University of Technology). Precursor solutions and particle collections may be made with sizes and lubricity for manual injection through a small gauge needle. Hydrophilic hydrogels crushed into spheroidal particles about 40 to about 100 microns diameter are small enough to be manually injected through a 30 gauge needle. A solvent with a high osmolarity and/or an osmolar agent to increase osmolarity may be used to ease passage of the particles/solutions through a needle.

Administration of a hydrogel and/or organogel and/or xerogel may be performed directly into the site of interest. Embodiments of the invention include administration at or near an eye. The structure of the mammalian eye can be divided into three main layers or tunics: the fibrous tunic, the vascular tunic, and the nervous tunic. The fibrous tunic, also known as the tunica fibrosa oculi, is the outer layer of the eyeball consisting of the cornea and sclera. The sclera extends from the cornea (the clear front section of the eye) to the optic nerve at the back of the eye. The sclera is a fibrous, elastic and protective tissue, composed of tightly packed collagen fibrils, containing about 70% water. Overlaying the fibrous tunic is the conjunctiva. The conjunctiva is a membrane that covers the sclera (white part of the eye) and lines the inside of the eyelids. The conjunctiva is typically divided into three parts: (a) Palpebral or tarsal conjunctivam which is the conjunctiva lining the eyelids; the palpebral conjunctiva is reflected at the superior fornix and the inferior fornix to become the bulbar conjunctiva; (b) Fornix conjunctiva: the conjunctiva where the inner part of the eyelids and the eyeball meet; and (c) Bulbar or ocular conjunctiva: The conjunctiva covering the eyeball, over the sclera. This region of the conjunctiva is bound tightly and moves with the eyeball movements. The conjunctiva effectively surrounds, covers, and adheres to the sclera. It is has cellular and connective tissue, is somewhat elastic, and can be removed, teased away, or otherwise taken down to expose a surface area of the sclera.

The vascular tunic, also known as the tunica vasculosa oculi, is the middle vascularized layer which includes the iris, ciliary body, and choroid. The choroid lies between the retina and sclera. The choroid contains blood vessels that supply the retinal cells with oxygen and remove the waste products of respiration. The choroid connects with the ciliary body toward the front of the eye and is attached to edges of the optic nerve at the back of the eye. The nervous tunic, also known as the tunica nervosa oculi, is the inner sensory which includes the retina. The retina contains the photosensitive rod and cone cells and associated neurons. The retina is a relatively smooth (but curved) layer. It does have two points at which it is different; the fovea and optic disc. The fovea is a dip in the retina directly opposite the lens, which is densely packed with cone cells. The fovea is part of the macula. The optic disc is a point on the retina where the optic nerve pierces the retina to connect to the nerve cells on its inside. The mammalian eye can also be divided into two main segments: the anterior segment and the posterior segment. The anterior segment consists of an anterior and posterior chamber.

The cornea and lens help to converge light rays to focus onto the retina. The lens, behind the iris, is a convex, springy disk which focuses light, through the second humour, onto the retina. It is attached to the ciliary body via a ring of suspensory ligaments known as the Zonule of Zinn. The iris, between the lens and the first humour, is a pigmented ring of fibrovascular tissue and muscle fibers. Light must first pass though the center of the iris, the pupil. Light enters the eye, passes through the cornea, and into the first of two humors, the aqueous humour. Approximately two-thirds of the total eyes refractive power comes from the cornea which has a fixed curvature. The aqueous humor is a clear mass which connects the cornea with the lens of the eye, helps maintain the convex shape of the cornea (necessary to the convergence of light at the lens) and provides the corneal endothelium with nutrients. The posterior segment is posterior to the crystalline lens and in front of the retina. It includes the anterior hyaloid membrane and the structures behind it, including the vitreous humor, retina, and optic nerve.

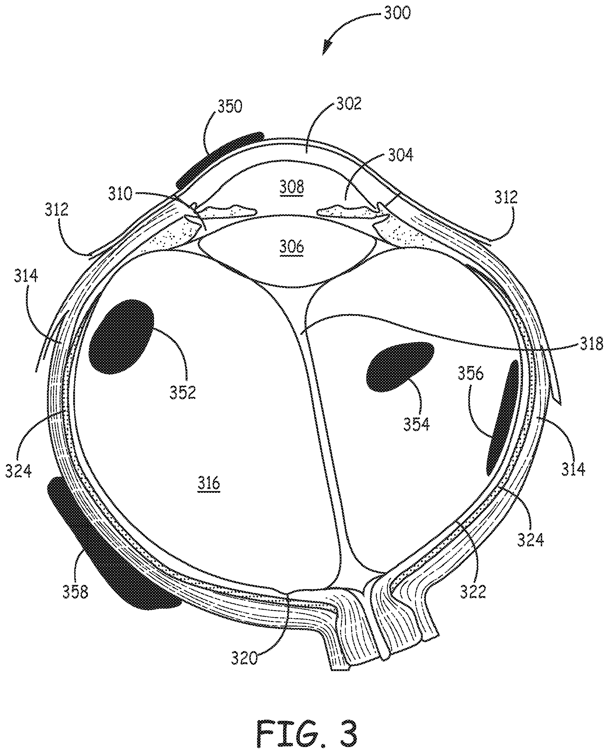

Figure 3 is a cross-section of eye 300 and depicts cornea 302 that is optically clear and allows light to pass iris 304 and penetrate lens 306. Anterior chamber 308 underlies cornea 302 and posterior chamber 310 lies between iris 304 and lens 306. Ciliary body 312 is connected to lens 306. Conjunctiva 312 which overlies sclera 314. The vitreous body 316 comprises the jelly-like vitreous humor, with hyaloid canal 318 being in the same. Fovea 320 is in the macula and retina 322 overlies choroid 324. Various points of delivery at eye 300 are depicted. One area is topically at 350. Another area is intravitreally as indicated at 352, 354, and 356; sites 352, 356 are out of the lens focal area, 356 is in contact with the inner edge of the eye, at the edge of the retina. Site 358 is outside of the eye and on the sclera. In use, for example a syringe, catheter (not shown) or other device is used to deliver the hydrogels 108, 110 or a hydrogel 108 and precursors 102. When precursors are delivered, they are chosen so they form hydrogel 110 *in situ* at the site of intended use. The therapeutic agents are released from the hydrogels.

Other sites may be chosen. Sites where drug delivery depots may be formed include the anterior chamber, posterior chamber, the vitreous, episcleral, subconjunctival, on a surface of a cornea or a conjunctiva, on a sclera, in a sclera, beneath a sclera, or between a sclera and subconjunctiva in a site under and contacting the conjunctiva, on or under the palpebral or tarsal conjunctivam, in an eyelid, superior fornix, inferior fornix, bulbar conjunctiva, and fornix conjunctiva. Further sites are in the choroid, between the choroid and sclera, between the retina and choroid, or a combination of the same.

The hydrogel may be placed at a site that is suited to deliver the agent for the pathology that is being treated. The choice of dose, size of implant, and position is affected by factors such as a time between repeat administrations, patient comfort or compliance, and dosage received at a target tissue. In general, back of the eye diseases can be treated with drugs utilizing, e.g., topical, systemic, intraocular and subconjunctival delivery routes. Systemic and topical (referring to eye drops and non-adherent materials) delivery modalities fall short in delivering therapeutic drug levels to treat posterior segment diseases: these methods of drug delivery encounter diffusion and drug dilution issues due to the inherent anatomical barriers of the intraocular and systemic systems, causing significant patient side effects (due to multiple daily dosing), poor bioavailability and compliance issues. Pericular drug delivery of an ophthalmic hydrogel implant using subconjunctival, scleral, suprachoroidal, retrobulbar or sub-Tenon's placement has the potential to offer a safer and enhanced drug delivery system to the retina compared to topical and systemic routes. For example; steroids like dexamethasone and triamicinolone acetonide may be mixed with the hydrogel precursor to form a sustained-release drug implant. The liquid hydrogel could then be injected in-situ into the sub-Tenon's capsule where it could deliver a constant or tunable release profile of the drug over a three to four month time period. The minimally invasive procedure could be performed in a doctor's office, or after a cataract operation under topical anesthesia, to treat chronic back of the eye diseases.

In some embodiments, a retractor is used to hold back eyelids, the user creatse a small buttonhole in the conjunctiva about 5-6 mm from the inferior/nasal limbus and dissect the conjunctiva down through Tenon's capsule, to the bare sclera. Next, a 23-gauge blunt cannula 86 (e.g., 15 mm in length) is inserted through the opening and the liquid drug implant is injected at the intended site of use. The cannula is then removed and the conjunctive is closed with a cauterization device. One advantage of a hydrogel implant having three dimensional integrity is that it will tend to resist cellular infiltration and be able to prevent the locally administered drug from being phagocytosed and cleared prematurely from the site. Instead, it stays in place until delivered. By way of contrast a microparticle, liposome, or pegylated protein tends to be rapidly cleared from the body by the reticuloendothelial system before being bioeffective.

Delivery of therapeutic amounts of a drug to the retina in posterior segment eye diseases remains a challenge. Although intravitreal injections into the vitreous cavity of anti-VEG F agents have shown promise to arrest and in some cases reverse chronic age-related diseases like macular degeneration, these techniques and procedures are not without risks and side effects. Intravitreal administration of therapeutic agents into the vitreous cavity can cause cataracts, endophthalmitis and retinal detachments. This form of therapy requires many patients to receive monthly intraocular injections of an anti-VEGF drug over a 12 month time period thus increasing the risk of infection, vitreous wicks and retinal detachments. Embodiments directed to an in situ hydrogel biodegradable drug implant that contains hydrogel particles will provide an effective alternative treatment for back of the eye diseases, and are expected to reduce the common side-effects associated with repeated intravitreal injections. For intravitreal implacement, for example, a hydrogel precursors and hydrogel particles are injected intravitrealy about 2.5 mm posterior to the limbus through a pars plana incision using a sub-retinal cannula, which may be made following dissecting-away or otherwise clearing the conjunctiva, as needed. A 25, 27 or 30 gauge sub-retinal cannula 94 (or other appropriate cannula) is then inserted and positioned intraocularly to the desired target site where the flowable precursors are introduced to form a hydrogel in-situ. The precursors then forms into an absorbable gel, adhering to the desired target site.

A drug depot of the in-situ hydrogel drug delivery implant may be designed for controlled, long term drug release ranging from, e.g., about one to about three months; and may optionally be directed to treatment of diseases of the posterior segment including, for example, age-related macular degeneration, diabetic retinopathy, diabetic macular edema, and the cystoid macular. The device can carry a drug payload of various types of therapeutic agents for various conditions, of which some include, for example, steroids, antibiotics, NSAIDS and/or antiangiogenic agents, or combinations thereof. The *in-situ* implant embodiments can improve the efficacy and pharmacokinetics of potent therapeutic agents in the treatment of chronic back of the eye diseases and minimize patient side effects in several ways. First, the implant can be placed in the vitreous cavity at a specific disease site, bypassing the topical or systemic routes and thereby increasing drug bioavailability. Secondly, the implant maintains local therapeutic concentrations at the specific target tissue site over an extended period of time. Thirdly, as compared to various conventional systems, the number of intravitreal injections would be substantially reduced, thereby reducing patient risk of infection, retinal detachment and transient visual acuity disturbances (white specks floating in the vitreous) that can occur until the drug in the vitreous migrates down toward the inferior wall of the eye and away from the portion of the central vitreous or macula. A bolus of conventionally-injected drugs forms in the vitreous body and displaces the vitreous humor until dispersed. Dispersion typically takes a significant amount of time since the vitreous humor is quite viscous. The bolus thus interferes with vision, particularly when it is moved around the eye in response to sudden accelerations, e.g., as the patient stands up or quickly turns the head.

The hydrogels may be formed in, on, or under scleral tissue either with or without the presence of the conjunctiva. The hydrogel may be adhesive to the sclera or other tissue where it is placed to promote drug diffusion through the intended tissue or to provide a stable depot to direct the therapeutic agents as required. In some embodiments, the conjunctiva of the eye may be removed, macerated, dissected away, or teased-free so that the tissue can be lifted away from the sclera to access a specific region of the sclera for implantation or injection of the hydrogel. In other embodiments, the hydrogel is injected in or on the choroid. A hydrogel is formed *in situ* that makes a layer on, and adheres, to the target site. In some embodiments the hydrogel is comprised of at least 50%, 75%, 80%, 90%, or 99% w/w water-soluble precursors (calculated by measuring the weight of the hydrophilic precursors and dividing by the weight of all precursors, so that the weight of water or solvents or non-hydrogel components is ignored) to enhance the non-adhesive properties of the hydrogel. In some embodiments, such hydrophilic precursors substantially comprise polyethylene oxides. In some embodiments, drugs to reduce tissue adherence mediated by biological mechanisms including cell mitosis, cell migration, or macrophage migration or activation, are included, e.g., anti-inflammatories, anti-mitotics, antibiotics, PACLITAXEL, MITOMYCIN, or taxols.

In some embodiments, the conjunctiva may be punctured or penetrated with a needle or catheter or trocar and precursors introduced into a space between the sclera and conjunctiva or other spaces in the eye. In some cases the conjunctiva may be punctured to access a natural potential space between the tissues that is filled by the precursors, for instance a supracchoroidal potential space. In other cases, a potential or actual space is created mechanically with a needle, trocar, spreader, or the like, that breaks the adherence between the tissue layers so that precursors may be introduced. The conjunctiva has enough elasticity to allow useful amounts of precursors to be introduced or forced into such natural or created spaces. Similarly, in the case of intravitreal hydrogel formation, relatively large volumes may also be used. Accordingly, in some cases, the amount is between about 0.001 to about 5 ml; artisans will immediately appreciate that all the ranges and values within the explicitly stated ranges are contemplated, e.g., about 1 ml, about 0.005, 0.01, 0.025, or 0.05 ml, or from 0.002 ml to about 1 or 2.5 ml.

Moreover, removal of a hydrogel, whether present intraocularly or periocularly, is also readily achieved using either a vitrectomy cutter if the implant is located in the vitreous cavity or a manual I/A syringe and cannula if the implant is located on the scleral surface or irrigation/aspiration handpiece. This contrasts with major surgical procedures needed for the removal of some conventional non-absorbable implants.

In some aspects, in-situ formation of the hydrogel lets the hydrogel gel or crosslink in place, so that it does not flow back out through the tract of the needle and diffuse extraocularly through the incision site upon the removal of the needle or cannula. A shape-stable hydrogel thus formed can effectively deliver the drug and advantageously can have well-controlled size, shape, and surface area. A small needle may be used to inject the materials since soluble or flowable precursors may be used instead of an already-formed material. By way of contrast, alternative materials that do not cross-link quickly and firmly upon introduction tend to flow back out of the incision. And materials that do not covalently cross-link are subject to creep or weeping as the material continually reorganizes and some or all of the material flows out.

### Drugs or other therapeutic agents for delivery

Therapeutic agents include, for example, agents for treating conditions that may result from inflammatory or abnormal vascular conditions, retinal vein occlusion, geographic atrophy, retinitis pigmentosa, retinoblastoma, etc.

Therapeutic agents may be those that are, e.g., antiangiogenic, anti-VEGF, blocks VEGFR1, blocks VEGFR2, blocks VEGFR3, anti-PDGF, anti-angiogenesis, Sunitinib, E7080, Takeda-6d, Tivozanib, Regorafenib, Sorafenib, Pazopanib, Axitinib, Nintedanib, Cediranib, Vatalanib, Motesanib, macrolides, sirolimus, everolimus, tyrosine kinase inhibitors (TKIs), Imatinib (GLEEVAC) gefinitib (IRESSA), toceranib (PALLADIA), Erlotinib (TARCEVA), Lapatinib (TYKERB) Nilotinib, Bosutinib Neratinib, lapatinib, Vatalanib, dasatinib, erlotinib, gefitinib, imatinib, lapatinib, lestaurtinib, nilotinib, semaxanib, , toceranib, vandetanib.

The therapeutic agent may comprise a macromolecule, for example an antibody or antibody fragment. The therapeutic macromolecule may comprise a VEGF inhibitor, for example ranibizumab, the active ingredient in the commercially available Lucentis™. The VEGF (Vascular Endothelial Growth Factor) inhibitor can cause regression of the abnormal blood vessels and improvement of vision when released into the vitreous humor of the eye. Examples of VEGF inhibitors include Lucentis™ (ranibizumab), Eylea™ (aflibercept or VEGF Trap), Avastin™ (bevacizumab), Macugen™ (pegaptanib). Platelet derived growth factor (PDGF) inhibitors may also be delivered, e.g., Fovista™, an anti-PGDF aptamer.

The therapeutic agent may comprise small molecules such as of a corticosteroid and analogues thereof. For example, the therapeutic corticosteroid may comprise one or more of trimacinalone, trimacinalone acetonide, dexamethasone, dexamethasone acetate, fluocinolone, fluocinolone acetate, or analogues thereof. Alternatively or in combination, the small molecules of therapeutic agent may comprise a tyrosine kinase inhibitor comprising one or more of axitinib, bosutinib, cediranib, dasatinib, erlotinib, gefitinib, imatinib, lapatinib, lestaurtinib, nilotinib, semaxanib, sunitinib, toceranib, vandetanib, or vatalanib, for example.

The therapeutic agent may comprise an anti-VEGF therapeutic agent. Anti-VEGF therapies and agents can be used in the treatment of certain cancers and in age-related macular degeneration. Examples of anti-VEGF therapeutic agents suitable for use in accordance with the embodiments described herein include one or more of monoclonal antibodies such as bevacizumab (Avastin™) or antibody derivatives such as ranibizumab (Lucentis™), or small molecules that inhibit the tyrosine kinases stimulated by VEGF such as lapatinib (Tykerb™), sunitinib (Sutent™), sorafenib (Nexavar™), axitinib, or pazopanib.

The therapeutic agent may comprise a therapeutic agent suitable for treatment of dry AMD such as one or more of Sirolimus™ (Rapamycin), Copaxone™ (Glatiramer Acetate), Othera™, Complement C5aR blocker, Ciliary Neurotrophic Factor, Fenretinide or Rheopheresis.

The therapeutic agent may comprise a therapeutic agent suitable for treatment of wet AMD such as one or more of REDD14NP (Quark), Sirolimus™ (Rapamycin), ATG003; Regeneron™ (VEGF Trap) or complement inhibitor (POT-4).

The therapeutic agent may comprise a kinase inhibitor such as one or more of bevacizumab (monoclonal antibody), BIBW 2992 (small molecule targeting EGFR/Erb2), cetuximab (monoclonal antibody), imatinib (small molecule), trastuzumab (monoclonal antibody), gefitinib (small molecule), ranibizumab (monoclonal antibody), pegaptanib (small molecule), sorafenib (small molecule), dasatinib (small molecule), sunitinib (small molecule), erlotinib (small molecule), nilotinib (small molecule), lapatinib (small molecule), panitumumab (monoclonal antibody), vandetanib (small molecule) or E7080 (targeting VEGFR2/VEGFR2, small molecule commercially available from Esai, Co.)

Therapeutic agents may include various classes of drugs. Drugs include, for instance, steroids, non-steroidal anti-inflammatory drugs (NSAIDS), anti-cancer drugs, antibiotics, an anti-inflammatory (e.g., Diclofenac), a pain reliever (e.g., Bupivacaine), a Calcium channel blocker (e.g., Nifedipine), an Antibiotic (e.g., Ciprofloxacin), a Cell cycle inhibitor (e.g., Simvastatin), a protein (e.g., Insulin). Therapeutic agents include classes of drugs including steroids, NSAIDS, antibiotics, pain relievers, inhibitors of vascular endothelial growth factor (VEGF), chemotherapeutics, anti-viral drugs, for instance. Examples of NSAIDS are Ibuprofen, Meclofenamate sodium, mefanamic acid, salsalate, sulindac, tolmetin sodium, ketoprofen, diflunisal, piroxicam, naproxen, etodolac, flurbiprofen, fenoprofen calcium, Indomethacin, celoxib, ketrolac, and nepafenac. The drugs themselves may be small molecules, proteins, RNA fragments, proteins, glycosaminoglycans, carbohydrates, nucleic acid, inorganic and organic biologically active compounds where specific biologically active agents include but are not limited to: enzymes, antibiotics, antineoplastic agents, local anesthetics, hormones, angiogenic agents, anti-angiogenic agents, growth factors, antibodies, neurotransmitters, psychoactive drugs, anticancer drugs, chemotherapeutic drugs, drugs affecting reproductive organs, genes, and oligonucleotides, or other configurations.

Therapeutic agents may include a protein or other water soluble biologics. These include peptides and proteins. The term protein, as used herein, refers to peptides of at least about 5000 Daltons. The term peptide, as used herein, refers to peptides of any size. The term oligopeptide refers to peptides having a mass of up to about 5000 Daltons. Peptides include therapeutic proteins and peptides, antibodies, antibody fragments, short chain variable fragments (scFv), growth factors, angiogenic factors, and insulin. Other water soluble biologics are carbohydrates, polysaccharides, nucleic acids, antisense nucleic acids, RNA, DNA, small interfering RNA (siRNA), and aptamers.

The therapeutic agents may be used as part of a method of treating the indicated condition or making a composition for treating the indicated condition. For example, AZOPT (a brinzolamide opthalmic suspension) may be used for treatment of elevated intraocular pressure in patients with ocular hypertension or open-angle glaucoma. BETADINE in a Povidone-iodine ophthalmic solution may be used for prepping of the periocular region and irrigation of the ocular surface. BETOPTIC (betaxolol HCl) may be used to lower intraocular pressure, or for chronic open-angle glaucoma and/or ocular hypertension. CILOXAN (Ciprofloxacin HCl opthalmic solution) may be used to treat infections caused by susceptible strains of microorganisms. NATACYN (Natamycin opthalmic suspension) may be used for treatment of fungal blepharitis, conjunctivitis, and keratitis. NEVANAC (Nepanfenac opthalmic suspension) may be used for treatment of pain and inflammation associated with cataract surgery. TRAVATAN (Travoprost ophthalmic solution) may be used for reduction of elevated intraocular pressure - open-angle glaucoma or ocular hypertension. FML FORTE (Fluorometholone ophthalmic suspension) may be used for treatment of corticosteroid-responsive inflammation of the palperbral and bulbar conjunctiva, cornea and anterior segment of the globe. LUMIGAN (Bimatoprost ophthalmic solution) may be used for reduction of elevated intraocular pressure - open-angle glaucoma or ocular hypertension. PRED FORTE (Prednisolone acetate) may be used for treatment of steroid-responsive inflammation of the palpebral and bulbar conjunctiva, cornea and anterior segment of the globe. PROPINE (Dipivefrin hydrochloride) may be used for control of intraocular pressure in chronic open-angle glaucoma. RESTASIS (Cyclosporine ophthalmic emulsion) may be used to increases tear production in patients, e.g., those with ocular inflammation associated with keratoconjunctivitis sicca. ALREX (Loteprednol etabonate ophthalmic suspension) may be used for temporary relief of seasonal allergic conjunctivitis. LOTEMAX (Loteprednol etabonate ophthalmic suspension) may be used for treatment of steroid-responsive inflammation of the palpebral and bulbar conjunctiva, cornea and anterior segment of the globe. MACUGEN (Pegaptanib sodium injection) may be used for Treatment of neovascular (wet) age-related macular degeneration. OPTIVAR (Azelastine hydrochloride) may be used for treatment of itching of the eye associated with allergic conjunctivitis. XALATAN (Latanoprost ophthalmic solution) may be used to reduce elevated intraocular pressure in patients, e.g., with open-angle glaucoma or ocular hypertension. BETIMOL (Timolol opthalmic solution) may be used for treatment of elevated intraocular pressure in patients with ocular hypertension or open-angle glaucoma. Latanoprost is the pro-drug of the free acid form, which is a prostanoid selective FP receptor agonist. Latanoprost reduces intraocular pressure in glaucoma patients with few side effects. Latanoprost has a relatively low solubility in aqueous solutions, but is readily soluble in organic solvents typically employed for fabrication of microspheres using solvent evaporation.

Further embodiments of therapeutic agents for delivery include those that specifically bind a target peptide *in vivo* to prevent the interaction of the target peptide with its natural receptor or other ligands. AVASTIN, for instance, is an antibody that binds VEGF. And AFLIBERCEPT is a fusion protein that includes portions of a VEGF receptor to trap VEGF. An IL-1 trap that makes use of the extracellular domains of IL-1 receptors is also known; the trap blocks IL-1 from binding and activating receptors on the surface of cells. Embodiments of agents for delivery include nucleic acids, e.g., aptamers. Pegaptanib (MACUGEN), for example, is a pegylated anti-VEGF aptamer. An advantage of the particle-and-hydrogel delivery process is that the aptamers are protected from the *in vivo* environment until they are released. Further embodiments of agents for delivery include macromolecular drugs, a term that refers to drugs that are significantly larger than classical small molecule drugs, i.e., drugs such as oligonucleotides (aptamers, antisense, RNAi), ribozymes, gene therapy nucleic acids, recombinant peptides, and antibodies.

One embodiment comprises extended release of a medication for allergic conjunctivitis. For instance, ketotifen, an antihistamine and mast cell stabilizer, may be provided in particles and released to the eye as described herein in effective amounts to treat allergic conjunctivitis. Seasonal Allergic Conjunctivitis (SAC) and Perennial Allergic Conjunctivitis (PAC) are allergic conjunctival disorders. Symptoms include itching and pink to reddish eyes. These two eye conditions are mediated by mast cells. Non-specific measures to ameliorate symptoms conventionally include: cold compresses, eyewashes with tear substitutes, and avoidance of allergens. Treatment conventionally consists of antihistamine mast cell stabilizers, dual mechanism anti-allergen agents, or topical antihistamines. Corticosteroids might be effective but, because of side effects, are reserved for more severe forms of allergic conjunctivitis such as vernal keratoconjunctivitis (VKC) and atopic keratoconjunctivitis (AKC).

Oxifloxacin is the active ingredient in VIGAMOX, which is a fluoroquinolone approved for use to treat or prevent ophthalmic bacterial infections. Dosage is typically one-drop of a 0.5% solution that is administered 3 times a day for a period of one-week or more. VKC and AKC are chronic allergic diseases where eosinophils, conjunctival fibroblasts, epithelial cells, mast cells, and/or TH2 lymphocytes aggravate the biochemistry and histology of the conjunctiva. VKC and AKC can be treated by medications used to combat allergic conjunctivitis. Permeation agents are agents and may also be included in a gel, hydrogel, organogel, xerogel, and biomaterials as described herein. These are agents that assist in permeation of a drug into an intended tissue. Permeation agents may be chosen as needed for the tissue, e.g., permeation agents for skin, permeation agents for an eardrum, permeation agents for an eye.

### Eye Disease States

The materials described herein may be used to deliver drugs or other therapeutic agents (e.g., imaging agents or markers) to eyes or tissues nearby. Some of the disease states are back-of-the-eye diseases. The term back-of-the eye disease is recognized by artisans in these fields of endeavor and generally refers to any ocular disease of the posterior segment that affects the vasculature and integrity of the retina, macula or choroid leading to visual acuity disturbances, loss of sight or blindness. Disease states of the posterior segment may result from age, trauma, surgical interventions, and hereditary factors. Some back-of-the-eye disease are; age-related macular degeneration (AMD) cystoid macular edema (CME), diabetic macular edema (DME), posterior uveitis, and diabetic retinopathy. Some back-of-the-eye diseases result from unwanted angiogenesis or vascular proliferation, such as macular degeneration or diabetic retinopathy. Drug treatment options for these and other conditions are further discussed elsewhere herein.

### Kits

Kits or systems for making hydrogels around hydrogel/xerogel particles may be prepared so that the hydrogel/xerogel particles comprising therapeutic agents are stored in the kit with precursors for making an enveloping hydrogel. Applicators may be used in combination with the same. The kits are manufactured using medically acceptable conditions and contain components that have sterility, purity and preparation that is pharmaceutically acceptable. Solvents/solutions may be provided in the kit or separately, or the components may be pre-mixed with the solvent. The kit may include syringes and/or needles for mixing and/or delivery. The kit or system may comprise components set forth herein.

### EXAMPLES

Some precursors are referred to by a nomenclature of *n*a*xx*K*pppfff*, where n is the number of arms, xx is the molecular weight (MW), ppp is the polymer, and fff is the functional end group. Thus 8a15KPEGSAP refers to an 8-armed Polyethylene glycol (PEG) with a MW of 15,000 g/mol = 15K PEG. Succinimidyl adipate is: SAP. Succinimidyl glutarate is SG. PEG refers to a polyethylene oxide and may or may not be terminated with an OH group.

### Example 1: Comparison of an in situ formed hydrogel coating on release kinetics of a therapeutic agent.

Materials Agent: monoclonal antibody (Mab) Bevacizumab (MW 149 kDa) entrapped in a hydrogel particle. 8a15KSS = 8 armed polyethylene glycol, MW 15,000 Da, each arm terminated with succinimidylsuccinate end groups. 8a20KNH2 = 8 armed polyethylene glycol, MW 20,000 Da, each arm terminated with free amine (not salt) end groups. 4a20KSAZ = 4 armed polyethylene glycol, MW 20,000 Da, each arm terminated with succinimidylazelate end groups. 8a20KNH3+ Cl- = 8 armed polyethylene glycol, MW 15,000 Da, each arm terminated with ammonium hydrochloride salt end groups. Particulate hydrogel-based protein delivery system: fast degrading 8a15KSS/8a20KNH2 was used for a rapid protein release from the inner hydrogel particles. Encapsulating Coating ("Envelope): in situ formed 4a20KSAZ/8a20KNH2 hydrogel.

### Methods

Spray dried powder of Bevacizumab (1.136g; 27% Active) was suspended in 3.5ml of 8a20KNH2 solution (11.4% in DMC), sonicated for 15 minutes then mixed with 3.5ml of 8a15KSS solution (8.6% in DMC) to form a bulk of 8a15KSS/8a20KNH2 organogel in DMC within 15 seconds. The organogel bulk was then cured at room temperature for 2 hours then reduced in particle size yielding a slurry of organogel particles in DMC. Organogel particles loaded with Bevacizumab (Bvcz) were then dried to form Bvcz xerogel loaded particles.

"No envelope" Individual samples of Bvcz Xerogel particles were mixed and rehydrated with 1% HA solution (4 hours) to form Bvcz hydrogel particles (10% Bvcz; 10% 8a15KSS/8a20KNH2; 80% of 1% HA solution). Samples were injected (using 21G2 needle) into tared vials and weighed (15 samples: 68.2; 37.1; 36.8; 37.6; 38.5; 43.7; 47.5; 28.0; 39.5; 39.3; 42.8; 42.4; 36.1; 70.5; 77.6mg). Individual samples were then released in 30ml of PBS (1x; pH 7.4) and pulled (3 samples/time point) at 1; 2; 3 and 9 days.

"Envelope" Bvcz Xerogel particles with in situ formed hydrogel envelope were prepared using syringes for rehydration and mixing. Syringe A contains Bvcz Xerogel particles (88.0 mg) and dry 4a20KSAZ polymer (15.3 mg); syringe B contains 0.4% HA solution (567.5 mg); syringe C with 211 mg of a 3.2% 8a20KNH3+ Cl- in pH 9.4 buffer (21.5 mg/ml Sodium tetraborate decahydrate; 7.1 mg/ml sodium phosphate dibasic). Bvcz Xerogel particles with in situ formed hydrogel envelope samples were prepared using individual kits (7 samples) by mixing syringes A and B then mixing the content with syringe C to form the envelope around the particles.

Individual samples were transferred to the PBS pH7.4 release media to determine the release kinetic profile of Bvcz and compare to the profile in the absence of the Envelope. Buffer was exchanged at 42, 68, 100, 119, 142 and 288 hrs.

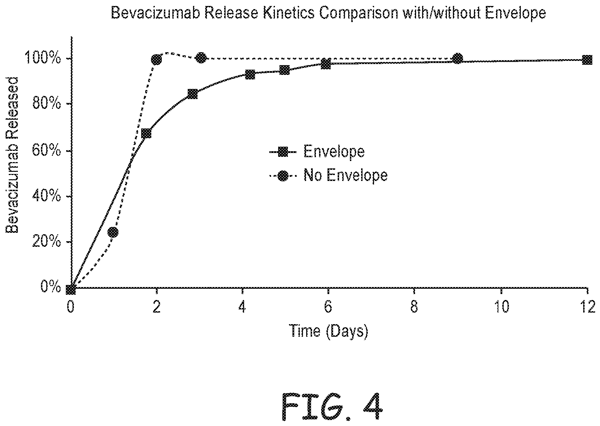

In vitro kinetic studies comparing sustained release of Bvcz from Xerogel particles with and without an "in situ formed" hydrogel envelope were then tested (Fig. 4). The coating envelope remained intact throughout the *in vitro* release experiment, so that the protein was forced to traverse through the envelope to reach the release media.

### Example 2: Intravitreal tolerability comparison of hydrogel particles with and without envelope.

Hydrogel formulations with and without encapsulating hydrogels ("envelopes") were tested in vivo. OTX-13 denotes particles with an envelope and OTX-14 denotes particles without an envelope.

Materials 8a5KSG = 8 armed polyethylene glycol, MW 5,000 Da, each arm terminated with succinimidylglutarate end groups. 8a10KSG = 8 armed polyethylene glycol, MW 10,000 Da, each arm terminated with succinimidylglutarate end groups. 8a5KNH2 = 8 armed polyethylene glycol, MW 5,000 Da, each arm terminated with a free amine (not salt) end groups. 4a20KSAZ = 4 armed polyethylene glycol, MW 20,000 Da, each arm terminated with succinimidylazelate end groups. 8a20KNH3+ Cl- = 8 armed polyethylene glycol, MW 15,000 Da, each arm terminated with ammonium hydrochloride salt end groups.

### Methods

Aliquots of 8a5KNH2 (30% in DMC) and 8a5KSG (30% in DMC) were mixed using syringes in a 1:1 ratio to form a bulk of 8a5KSG/8a5KNH2 organogel in DMC. The organogel bulk is then cured at room temperature for 2 hours then reduced in particle size yielding to a slurry of organogel particles in DMC. The resulting blank organogel particles in DMC were then dried to form 8a5KSG/8a5KNH2 xerogel blank particles. Aliquots of 8a5KNH2 (20% in DMC) and 8a10KSG (40% in DMC) were mixed using syringes in a 1:1 ratio to form a bulk of 8a10KSG/8a5KNH2 organogel in DMC. The organogel bulk was then cured at room temperature for 2 hours then reduced in particle size yielding to a slurry of organogel particles in DMC. The resulting blank organogel particles in DMC were then dried to form 8a10KSG/8a5KNH2 xerogel blank particles.

OTX-14 (No Envelope) was prepared by weighing 8a5KSG/8a5KNH2 (72.8mg) and 8a10KSG/8a5KNH2 (48.5mg) xerogel blank particles in syringe A, weighing Provisc (519.3 mg; 1% HA) in syringe B then mixing syringe A and B where OTX-14 was ready for intravitreal injection.

OTX-13 (Envelope) was prepared by weighing 8a5KSG/8a5KNH2 (58.7mg) and 8a10KSG/8a5KNH2 (42.4mg) xerogel blank particles as well as 4a20KSAZ (16.2mg) in syringe A; filling diluted PROVISC (664.3 mg; 0.41% HA in PBS pH7.4) in syringe B; filling 8a20KNH3+ Cl- (257 mg; 3.2% in pH 10.0 buffer: 21.5 mg/ml Sodium tetraborate decahydrate; 7.1 mg/ml sodium phosphate dibasic) in syringe C. Individual injections were prepared by mixing syringe A with B for 1 minute then mixing with syringe C for 10 seconds to initiate the reaction. At this point the mixture is transferred to 100ul syringe for intravitreal injection. 25 ul were injected. Injected rabbits were sacrificed at day 28 and day 56, eyes harvested and analyzed by histopathology. Tissues were scored on a semi-quantitative scale from 0-5 for any abnormalities.

| **TABLE 1** | |
|---|---|
| **Score** | **Description** |
| 0 | No change; normal |
| 1 | Rare foci of change; Minimal |
| 2 | Mild diffuse change or more pronounced focal change |
| 3 | Moderate diffuse change |
| 4 | Marked diffuse change |
| 5 | Severe diffuse change |

Intravitreal injection of OTX-13 resulted in slightly decreased inflammation in the vitreous chamber compared to OTX-14 at both time points. Both formulations resulted in similar typically minimal inflammation in the vitreous chamber around the injected test material, or observed as scattered macrophages within the vitreous chamber. Rarely, such inflammation extended minimally into the retina or sclera. OTX-13 showed a markedly lower inflammation as compared to OTX-14 around the retina (Table 2: 0.02-0.03 as compared to 0.1-0.2).

**TABLE 2**

| **Time (Days)** | Inflammation Score (0-5) | | | | | | | |
|---|---|---|---|---|---|---|---|---|
| | **Vitreous** | | | | **Retina/Sclera** | | | |
| | **OTX-13** | | **OTX-14** | | **OTX-13** | | **OTX-14** | |
| | **Mean** | **Stdev** | **Mean** | **Stdev** | **Mean** | **Stdev** | **Mean** | **Stdev** |
| **28** | **0.9** | 0.3 | **1.2** | 0.2 | **0.03** | 0.05 | **0.2** | 0.3 |
| **56** | **1** | 0.1 | **1.1** | 0.1 | **0.02** | 0.04 | **0.1** | 0.1 |

Intravitreal injection of OTX-13 and OTX-14 resulted in similar typically minimal fibrosis around the implanted material in the vitreous chamber. The mean score for OTX-13 is slightly decreased compared to OTX-14 at both time points.

Intravitreal injection of OTX-13 and OTX-14 resulted in similar typically minimal epithelial hyperplasia and inflammation in epithelium just in front of the ora serrata. The mean score for OTX-13 were slightly different compared to OTX-14 at both time points.

**TABLE 3**

| **Time (Days)** | Tissue Reaction Score (0-5) | | | | | | | |
|---|---|---|---|---|---|---|---|---|
| | **Fibrosis near implant** | | | | **Hyperplasia-Ora serrata** | | | |
| | **OTX-13** | | **OTX-14** | | **OTX-13 OTX-14** | | | |
| | **Mean** | **Stdev** | **Mean** | **Stdev** | **Mean** | **Stdev** | **Mean** | **Stdev** |
| **28** | **0.1** | 0.1 | **0.1** | 0.1 | **0.1** | 0.1 | **0.1** | 0.1 |
| **56** | **0.1** | 0.1 | **0.3** | 0.1 | **0.5** | 0.2 | **0.4** | 0.3 |

## Claims

1. A biomedical sustained release system for use as a medicament in the treatment of an eye disease, especially as an eye implant comprising
a collection of particles that comprise a first biodegradable material that is a hydrogel or a xerogel made of hydrophilic materials and a therapeutic agent, with the particles, before biodegradation of the first material, having a rate of release for the therapeutic agent as measured in physiological solution, and
a second material that is a hydrogel or xerogel that at least partially coats the collection of particles wherein the hydrogel made of the second material delays the rate of release of the agent by no more than 20% as measured at the 50% w/w release of the agent.

2. The system of claim 1 wherein a solids content of the second material is lower than a solids content of the particles, and the solids content of the second material is in a range from about 2.5% to about 20% w/w.

3. The system of claim 1 or 2 wherein the hydrogel made of the second material has a molecular weight between crosslinks that is at least 3000 Da.

4. The system of any of claims 2-3 wherein the delay of the rate of the release as measured at the 50% w/w release of the agent is no more than 10%.

5. The system of any of claims 2-4 wherein the therapeutic agent is a protein.

6. The system of any of claims 2-5 wherein the first material comprises a first precursor that comprises first functional groups and a second precursor that comprises second functional groups, with the first functional groups and the second functional groups forming covalent crosslinks, and the second material comprises a third precursor that comprises third functional groups and a fourth precursor that comprises fourth functional groups, with the third functional groups and the fourth functional groups forming covalent crosslinks.

7. The system of claim 6 wherein the first through fourth functional groups, before reaction, are selected from the group consisting of electrophilic groups and nucleophilic groups.

8. The system of claim 6 or 7 wherein the first through fourth precursors are, before being covalently crosslinked, water soluble.

9. A kit for making the biomedical sustained release system of any of claim 1-8 comprising hydrogel or xerogel particles comprising therapeutic agents and precursors for making an enveloping hydrogel.

10. The kit of claim 9 wherein the second hydrogel is free of the therapeutic agent until such time as the agent diffuses from the particles into the second hydrogel.

11. The kit of claim 9 or 10 wherein the particles have a diameter that is within a range from 1 to 100 µm.

12. The kit of any of claims 9-11, further comprising an applicator.

13. The kit of any of claims 9-11 for use as a medicament, especially in the treatment of an eye disease.

14. The kit of claim 12 for delivering a medicament, especially in the treatment of an eye disease.

## Patentansprüche

1. Biomedizinisches Retardsystem zur Verwendung als Medikament bei der Behandlung einer Augenerkrankung, insbesondere als Augenimplantat, umfassend
eine Sammlung von Teilchen, die ein erstes biologisch abbaubares Material, bei dem es sich um ein aus hydrophilen Materialien und einem therapeutischen Mittel hergestelltes Hydrogel oder Xerogel handelt, wobei die Teilchen vor dem biologischen Abbau des ersten Materials eine Freisetzungsrate für das therapeutische Mittel, bezogen auf die Messung in physiologischer Lösung, aufweisen, und
ein zweites Material, bei dem es sich um ein die Sammlung von Teilchen zumindest teilweise ummantelndes Hydrogel oder Xerogel handelt, wobei das aus dem zweiten Material hergestellte Hydrogel die Freisetzungsrate des Mittels um nicht mehr als 20 %, bezogen auf die Messung bei 50%iger (w/w) Freisetzung des Mittels, verzögert.

2. System nach Anspruch 1, wobei ein Feststoffgehalt des zweiten Materials niedriger ist als ein Feststoffgehalt der Teilchen und wobei der Feststoffgehalt des zweiten Materials im Bereich von etwa 2,5 % bis etwa 20 % (w/w) liegt.

3. System nach Anspruch 1 oder 2, wobei das aus dem zweiten Material hergestellte Hydrogel zwischen den Vernetzungen ein Molekulargewicht aufweist, das mindestens 3000 Da beträgt.

4. System nach einem der Ansprüche 2-3, wobei die Verzögerung der Freisetzungsrate, bezogen auf die Messung bei 50%iger (w/w) Freisetzung des Mittels, nicht mehr als 10 % beträgt.

5. System nach einem der Ansprüche 2-4, wobei es sich bei dem therapeutischen Mittel um ein Protein handelt.

6. System nach einem der Ansprüche 2-5, wobei das erste Material einen erste funktionelle Gruppen umfassenden ersten Vorläufer und einen zweite funktionelle Gruppen umfassenden zweiten Vorläufer umfasst, wobei die ersten funktionellen Gruppen und die zweiten funktionellen Gruppen kovalente Vernetzungen bilden, und das zweite Material einen dritte funktionelle Gruppen umfassenden dritten Vorläufer und einen vierte funktionelle Gruppen umfassenden vierten Vorläufer umfasst, wobei die dritten funktionellen Gruppen und die vierten funktionellen Gruppen kovalente Vernetzungen bilden.

7. System nach Anspruch 6, wobei die ersten bis vierten funktionellen Gruppen vor der Reaktion aus der Gruppe bestehend aus elektrophilen Gruppen und nukleophilen Gruppen ausgewählt sind.

8. System nach Anspruch 6 oder 7, wobei die ersten bis vierten Vorläufer vor der kovalenten Vernetzung wasserlöslich sind.

9. Kit zum Herstellen eines biomedizinischen Retardsystems nach einem der Ansprüche 1-8, umfassend Hydrogel- oder Xerogelteilchen, die therapeutische Mittel und Vorläufer zum Herstellen eines Umhüllungshydrogels umfassen.

10. Kit nach Anspruch 9, wobei das zweite Hydrogel solange frei von dem therapeutischen Mittel ist, bis das Mittel aus den Teilchen in das zweite Hydrogel diffundiert.

11. Kit nach Anspruch 9 oder 10, wobei die Teilchen einen Durchmesser, der im Bereich von 1 bis 100 µm liegt, aufweisen.

12. Kit nach einem der Ansprüche 9-11, ferner umfassend einen Applikator.

13. Kit nach einem der Ansprüche 9-11 zur Verwendung als Medikament, insbesondere bei der Behandlung einer Augenerkrankung.

14. Kit nach Anspruch 12 zur Zuführung eines Medikaments, insbesondere bei der Behandlung einer Augenerkrankung.

## Revendications

1. Système biomédical à libération prolongée pour son utilisation en tant que médicament dans le traitement d'une maladie de l'œil, notamment en tant qu'implant de l'œil, comprenant :
un recueil de particules qui comprennent un premier matériau biodégradable qui est un hydrogel ou un xérogel réalisé en matériaux hydrophiles et un agent thérapeutique, les particules, avant biodégradation du premier matériau, ayant une vitesse de libération pour l'agent thérapeutique telle que mesurée dans une solution physiologique, et
un second matériau qui est un hydrogel ou xérogel qui enrobe au moins partiellement le recueil de particules, dans lequel l'hydrogel réalisé en le second matériau retarde la vitesse de libération de l'agent de pas plus de 20 % telle que mesurée à la libération de 50 % p/p de l'agent.

2. Système selon la revendication 1, dans lequel une teneur en matières sèches du second matériau est inférieure à une teneur en matières sèches des particules, et la teneur en matières sèches du second matériau est dans une plage d'environ 2,5 % à environ 20 % p/p.

3. Système selon la revendication 1 ou 2, dans lequel l'hydrogel réalisé en le second matériau a un poids moléculaire entre des réticulations qui est d'au moins 3000 Da.

4. Système selon l'une quelconque des revendications 2 et 3, dans lequel le retard de la vitesse de libération telle que mesurée à la libération de 50 % p/p de l'agent n'est pas plus de 10 %.

5. Système selon l'une quelconque des revendications 2 à 4, dans lequel l'agent thérapeutique est une protéine.

6. Système selon l'une quelconque des revendications 2 à 5, dans lequel le premier matériau comprend un premier précurseur qui comprend des premiers groupes fonctionnels et un deuxième précurseur qui comprend des deuxièmes groupes fonctionnels, les premiers groupes fonctionnels et les deuxièmes groupes fonctionnels formant des réticulations covalentes, et le second matériau comprend un troisième précurseur qui comprend des troisièmes groupes fonctionnels et un quatrième précurseur qui comprend des quatrièmes groupes fonctionnels, les troisièmes groupes fonctionnels et les quatrièmes groupes fonctionnels formant des réticulations covalentes.

7. Système selon la revendication 6, dans lequel les premiers à quatrièmes groupes fonctionnels, avant réaction, sont choisis dans le groupe constitués de groupes électrophiles et de groupes nucléophiles.

8. Système selon la revendication 6 ou 7, dans lequel les premier à quatrième précurseurs, avant d'être réticulés de façon covalente, sont solubles dans l'eau.

9. Nécessaire pour réaliser le système biomédical à libération prolongée de l'une quelconque des revendications 1 à 8 comprenant des particules hydrogel ou de xérogel comprenant des agents thérapeutiques et des précurseurs pour réaliser un hydrogel d'enveloppement.

10. Nécessaire selon la revendication 9, dans lequel le second hydrogel est dépourvu de l'agent thérapeutique jusqu'à ce que l'agent se diffuse depuis les particules dans le second hydrogel.

11. Nécessaire selon la revendication 9 ou 10, dans lequel les particules ont un diamètre qui est dans une plage de 1 à 100 µm.

12. Nécessaire selon l'une quelconque des revendications 9 à 11, comprenant en outre un applicateur.

13. Nécessaire selon l'une quelconque des revendications 9 à 11, pour son utilisation en tant que médicament, notamment dans le traitement d'une maladie de l'œil.

14. Nécessaire selon la revendication 12 pour administrer un médicament, notamment dans le traitement d'une maladie de l'œil.
